# EUROPÄISCHE PATENTSCHRIFT

(11) **EP 0 852 623 B1**
(45) Veröffentlichungstag und Bekanntmachung des Hinweises auf die Patenterteilung: **30.11.2005**
(21) Anmeldenummer: 96932563.8
(22) Anmeldetag: 18.09.1996
(51) Int. Cl.: C12N 15/31, C12N 15/70, C12N 1/21, C12Q 1/68, C07K 14/22, C07K 16/12, A61K 39/095, A61K 39/40, G01N 33/569

(54) **NUCLEINSÄURE-MOLEKÜLE CODIEREND PROTEINE, DIE DIE ADHÄSION VON NEISSERIA-ZELLEN AN HUMANE ZELLEN VERMITTELN**
NUCLEIC ACID MOLECULES WHICH CODE FOR PROTEINS WHICH MEDIATE THE ADHESION OF NEISSERIA CELLS TO HUMAN CELLS
MOLECULES D'ACIDE NUCLEIQUE CODANT DES PROTEINES JOUANT UN ROLE DE MEDIATION DANS L'ADHESION DE CELLULES DE BACTERIES NEISSERIA A DES CELLULES HUMAINES

(30) Priorität: 18.09.1995 DE 19534579
(43) Veröffentlichungstag der Anmeldung: 15.07.1998
(73) Patentinhaber: MAX-PLANCK-GESELLSCHAFT ZUR FÖRDERUNG DER WISSENSCHAFTEN E.V., 14195 Berlin (DE)
(72) Erfinder: MEYER, Thomas, F., D-72076 Tübingen (DE); RUDEL, Thomas, La Jolla, CA 92122 (US); SCHEUERPFLUG, Ina, D-14193 Berlin (DE); FISCHER, Eckhard, D-72076 Tübingen (DE); MAIER, Jürgen, D-73257 Köngen (DE); EICKERNJÄGER, Sandra, 13357 Berlin (DE); SCHWAN, Thomas, 12161 Berlin (DE)
(74) Vertreter: Vossius & Partner
(86) Internationale Anmeldenummer: PCT/EP1996/004092
(87) Internationale Veröffentlichungsnummer: WO 1997/011181

(56) Entgegenhaltungen:
- WO-A-92/13871
- DE-C- 4 336 530
- CLINICAL MICROBIOLOGY REVIEWS, Bd. 8, Nr. 3, Juli 1995, Seiten 376-388, XP000644345 NASSIF X. AND SO M.: "Interaction of pathogenic Neisseriae with nonphagocytic cells"
- PNAS, U.S.A., Bd. 87, Nr. 1, Januar 1990, Seiten 333-337, XP002024933 PARUCHURI D. ET AL.: "Identification and characterization of a Neisseria gonorrhoeae gene encoding a glycolipid-binding adhesin"

### SEQUENZPROTOKOLL

(1) ALLGEMEINE ANGABEN:
   (i) ANMELDER:
      (A) NAME: Max-Planck-Gesellschaft zur Förderung der Wissenschaften e.V.
      (B) STRASSE: keine
      (C) ORT: Berlin
      (E) LAND: Deutschland
      (F) POSTLEITZAHL: keiner
   (ii) BEZEICHNUNG DER ERFINDUNG: Nucleinsäuremoleküle codierend Proteine, die die Adhäsion von Neisseria-Zellen an humane Zellen vermitteln
   (iii) ANZAHL DER SEQUENZEN: 4
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
   (vi) DATEN DER URANMELDUNG:
      (A) ANMELDENUMMER: DE 19534579.7
      (B) ANMELDETAG: 18-SEP-1995
(2) ANGABEN ZU SEQ ID NO: 1:
   (i) SEQUENZKENNZEICHEN:
      (A) LÄNGE: 3287 Basenpaare
      (B) ART: Nucleotid
      (C) STRANGFORM: Doppelstrang
      (D) TOPOLOGIE: linear
   (ii) ART DES MOLEKÜLS: Genom-DNA
   (iii) HYPOTHETISCH: NEIN
   (iv) ANTISENSE: NEIN
   (vi) URSPRÜNLICHE HERKUNFT:
      (A) ORGANISMUS: Neisseria gonorrheae
      (B) STAMM: MS11
   (vii) UNMITTELBARE HERKUNFT:
      (A) BIBLIOTHEK: genomische Bibliothek in pBA
      (B) CLON(E): H1967/pES25
   (ix) MERKMAL:
      (A) NAME/SCHLÜSSEL: CDS
      (B) LAGE:136..447
   (ix) MERKMAL:
      (A) NAME/SCHLÜSSEL: CDS
      (B) LAGE:583..1542
   (ix) MERKMAL:
      (A) NAME/SCHLÜSSEL: CDS
      (B) LAGE:1585..3111
   (xi) SEQUENZBESCHREIBUNG: SEQ ID NO: 1:
   CGGCGCAAAC GGCGGACGCT GCTGTTAGCC CCGCTTGAAA CAAATGCCGT CTGAACGCCA      60
   CTTCAGACGG CATTTTTATA ATAAGGCGCT GTCCTAGATA ACTAGGGAAA TTCAAATTAA     120
   GTTAGAATTA TCCCT ATG AGA AAA AGC CGT CTA AGC CGG TAT AAA CAA AAT     171
                    Met Arg Lys Ser Arg Leu Ser Arg Tyr Lys Gln Asn
                     1               5                  10
   AAA CTC ATT GAA CTG TTT GTC GCA GGC GTA ACT GCA AGA ACA GCA GCA      219
   Lys Leu Ile Glu Leu Phe Val Ala Gly Val Thr Ala Arg Thr Ala Ala
            15                  20                  25
   GAG CCT GAC AGC ATT GTT TAT ACG GAT TGT TAT CGT CGC TAT GAT GTA      267
   Glu Pro Asp Ser Ile Val Tyr Thr Asp Cys Tyr Arg Arg Tyr Asp Val
        30                  35                  40
   TTG GAT GCG GGC GAA TTT AGC CAT TTC CGT ATC AAT CAC AGC ACA CAT      315
   Leu Asp Ala Gly Glu Phe Ser His Phe Arg Ile Asn His Ser Thr His
    45                  50                  55                  60
   TTT GCC GAA CGA CAA AAC CAT ATT AAT GGA ATT GGG AAC TTT TGG AAC      363
   Phe Ala Glu Arg Gln Asn His Ile Asn Gly Ile Gly Asn Phe Trp Asn
                    65                  70                  75
   CGG GCA AAA CGT CAT TTA CGC AAG TTT GAC GGC ATT CCC AAA GAG CAT      411
   Arg Ala Lys Arg His Leu Arg Lys Phe Asp Gly Ile Pro Lys Glu His
                80                  85                  90
   TTT GAG CCG TAT TTA AAG GAG TGC GAA CGG CGT TTT TAACAACAGT           457
   Phe Glu Pro Tyr Leu Lys Glu Cys Glu Arg Arg Phe
            95                 100
   GAGATAAAAG TTCTTGTTCC ATTTTAAAAC AATTAGTAAA ATCGAGTTTA TCCTAGTTGT     517
   CCAGGACGGC CCCTAATTTA TTTACAATTT TGATACAATT TGTTTTTCAT CAAAGGAGAA     577
   AATCT ATG CGG GCA CGG CTG CTG ATA CCT ATT CTT TTT TCG GTT TTT        624
         Met Arg Ala Arg Leu Leu Ile Pro Ile Leu Phe Ser Val Phe
           1               5                  10
   ATT TTA TCC GCC TGC GGG ACA CTG ACA GGT ATT CCA TCG CAT GGC GGA      672
   Ile Leu Ser Ala Cys Gly Thr Leu Thr Gly Ile Pro Ser His Gly Gly
    15                  20                  25                  30
   GGC AAA CGC TTC GCG GTC GAA CAA GAA CTT GTG GCC GCT TCT GCC AGA      720
   Gly Lys Arg Phe Ala Val Glu Gln Glu Leu Val Ala Ala Ser Ala Arg
                    35                  40                  45
   GCT GCC GTT AAA GAC ATG GAT TTA CAG GCA TTA CAC GGA CGA AAA GTT      768
   Ala Ala Val Lys Asp Met Asp Leu Gln Ala Leu His Gly Arg Lys Val
                50                  55                  60
   GCA TTG TAC ATT GCA ACT ATG GGC GAC CAA GGT TCA GGC AGT TTG ACA      816
   Ala Leu Tyr Ile Ala Thr Met Gly Asp Gln Gly Ser Gly Ser Leu Thr
            65                  70                  75
   GGG GGT CGC TAC TCC ATT GAT GCA CTG ATT CGC GGC GAA TAC ATA AAC      864
   Gly Gly Arg Tyr Ser Ile Asp Ala Leu Ile Arg Gly Glu Tyr Ile Asn
        80                  85                  90
   AGC CCT GCC GTC CGC ACC GAT TAC ACC TAT CCG CGT TAC GAA ACC ACC      912
   Ser Pro Ala Val Arg Thr Asp Tyr Thr Tyr Pro Arg Tyr Glu Thr Thr
    95                 100                 105                 110
   GCT GAA ACA ACA TCA GGC GGT TTG ACG GGT TTA ACC ACT TCT TTA TCT      960
   Ala Glu Thr Thr Ser Gly Gly Leu Thr Gly Leu Thr Thr Ser Leu Ser
                   115                 120                 125
   ACA CTT AAT GCC CCT GCA CTC TCG CGC ACC CAA TCA GAC GGT AGC GGA     1008
   Thr Leu Asn Ala Pro Ala Leu Ser Arg Thr Gln Ser Asp Gly Ser Gly
               130                 135                 140
   AGT AGG AGC AGT CTG GGC TTA AAT ATT GGC GGG ATG GGG GAT TAT CGA     1056
   Ser Arg Ser Ser Leu Gly Leu Asn Ile Gly Gly Met Gly Asp Tyr Arg
           145                 150                 155
   AAT GAA ACC TTG ACG ACC AAC CCG CGC GAC ACT GCC TTT CTT TCC CAC     1104
   Asn Glu Thr Leu Thr Thr Asn Pro Arg Asp Thr Ala Phe Leu Ser His
       160                 165                 170
   TTG GTA CAG ACC GTA TTT TTC CTG CGC GGC ATA GAC GTT GTT TCT CCT     1152
   Leu Val Gln Thr Val Phe Phe Leu Arg Gly Ile Asp Val Val Ser Pro
   175                 180                 185                 190
   GCC AAT GCC GAT ACA GAT GTG TTT ATT AAC ATC GAC GTA TTC GGA ACG     1200
   Ala Asn Ala Asp Thr Asp Val Phe Ile Asn Ile Asp Val Phe Gly Thr
                   195                 200                 205
   ATA CGC AAC AGA ACC GAA ATG CAC CTA TAC AAT GCC GAA ACA CTG AAA     1248
   Ile Arg Asn Arg Thr Glu Met His Leu Tyr Asn Ala Glu Thr Leu Lys
               210                 215                 220
   GCC CAA ACA AAA CTG GAA TAT TTC GCA GTA GAC AGA ACC AAT AAA AAA     1296
   Ala Gln Thr Lys Leu Glu Tyr Phe Ala Val Asp Arg Thr Asn Lys Lys
           225                 230                 235
   TTG CTC ATC AAA CCC AAA ACC AAT GCG TTT GAA GCT GCC TAT AAA GAA     1344
   Leu Leu Ile Lys Pro Lys Thr Asn Ala Phe Glu Ala Ala Tyr Lys Glu
       240                 245                 250
   AAT TAC GCA TTG TGG ATG GGG CCG TAT AAA GTA AGC AAA GGA ATC AAA      1392
   Asn Tyr Ala Leu Trp Met Gly Pro Tyr Lys Val Ser Lys Gly Ile Lys
   255                 260                 265                 270
   CCG ACG GAA GGA TTA ATG GTC GAT TTC TCC GAT ATC CGG CCA TAC GGC      1440
   Pro Thr Glu Gly Leu Met Val Asp Phe Ser Asp Ile Arg Pro Tyr Gly
               275                 280                 285
   AAT CAT ACG GGT AAC TCC GCC CCA TCC GTA GAG GCT GAT AAC AGT CAT      1488
   Asn His Thr Gly Asn Ser Ala Pro Ser Val Glu Ala Asp Asn Ser His
           290                 295                 300
   GAG GGG TAT GGA TAC AGC GAT GAA GCA GTG CGA CAA CAT AGA CAA GGG      1536
   Glu Gly Tyr Gly Tyr Ser Asp Glu Ala Val Arg Gln His Arg Gln Gly
       305                 310                 315
   CAA CCT TGATTCACAC TGCCATAACC GCTTGCTGCC AAGGAAAACA AA ATG AAT       1590
   Gln Pro                                                Met Asn
       320                                                     1
   TTG CCT ATT CAA AAA TTC ATG ATG CTG TTT GCA GCG GCA ATA TCG TTG      1638
   Leu Pro Ile Gln Lys Phe Met Met Leu Phe Ala Ala Ala Ile Ser Leu
           5                  10                  15
   CTG CAA ATC CCC ATT AGT CAT GCG AAC GGT TTG GAT GCC CGT TTG CGC      1686
   Leu Gln Ile Pro Ile Ser His Ala Asn Gly Leu Asp Ala Arg Leu Arg
        20                  25                  30
   GAT GAT ATG CAG GCA AAA CAC TAC GAA CCG GGT GGC AAA TAC CAT CTG      1734
   Asp Asp Met Gln Ala Lys His Tyr Glu Pro Gly Gly Lys Tyr His Leu
    35                  40                  45                  50
   TTC GGT AAT GCT CGC GGC AGT GTT AAA AAT CGG GTT TGC GCC GTC CAA      1782
   Phe Gly Asn Ala Arg Gly Ser Val Lys Asn Arg Val Cys Ala Val Gln
                    55                  60                  65
   ACA TTT GAT GCA ACT GCG GTC GGC CCC ATA CTG CCT ATT ACA CAC GAA      1830
   Thr Phe Asp Ala Thr Ala Val Gly Pro Ile Leu Pro Ile Thr His Glu
                70                  75                  80
   CGG ACA GGG TTT GAA GGC ATT ATC GGT TAT GAA ACC CAT TTT TCA GGA      1878
   Arg Thr Gly Phe Glu Gly Ile Ile Gly Tyr Glu Thr His Phe Ser Gly
            85                  90                  95
   CAC GGA CAC GAA GTA CAC AGT CCG TTC GAT AAT CAT GAT TCA AAA AGC      1926
   His Gly His Glu Val His Ser Pro Phe Asp Asn His Asp Ser Lys Ser
       100                 105                 110
   ACT TCT GAT TTC AGC GGC GGC GTA GAC GGC GGT TTT ACC GTT TAC CAA      1974
   Thr Ser Asp Phe Ser Gly Gly Val Asp Gly Gly Phe Thr Val Tyr Gln
   115                 120                 125                 130
   CTT CAT CGG ACA GGG TCG GAA ATA CAT CCC GCA GAC GGA TAT GAC GGG      2022
   Leu His Arg Thr Gly Ser Glu Ile His Pro Ala Asp Gly Tyr Asp Gly
               135                 140                 145
   CCT CAA GGC GGC GGT TAT CCG GAA CCA CAA GGG GCA AGG GAT ATA TAC        2070
   Pro Gln Gly Gly Gly Tyr Pro Glu Pro Gln Gly Ala Arg Asp Ile Tyr
               150                 155                 160
   AGC TAC CAT ATC AAA GGA ACT TCA ACC AAA ACA AAG ATA AAC ACT GTT        2118
   Ser Tyr His Ile Lys Gly Thr Ser Thr Lys Thr Lys Ile Asn Thr Val
           165                 170                 175
   CCG CAA GCC CCT TTT TCA GAC CGC TGG CTA AAA GAA AAT GCC GGT GCC        2166
   Pro Gln Ala Pro Phe Ser Asp Arg Trp Leu Lys Glu Asn Ala Gly Ala
       180                 185                 190
   GCT TCC GGT TTT CTC AGC CGT GCG GAT GAA GCA GGA AAA CTG ATA TGG        2214
   Ala Ser Gly Phe Leu Ser Arg Ala Asp Glu Ala Gly Lys Leu Ile Trp
   195                 200                 205                 210
   GAA AAC GAC CCC GAT AAA AAT TGG CGG GCT AAC CGT ATG GAT GAT ATT        2262
   Glu Asn Asp Pro Asp Lys Asn Trp Arg Ala Asn Arg Met Asp Asp Ile
                   215                 220                 225
   CGC GGC ATC GTC CAA GGT GCG GTT AAT CCT TTT TTA ACG GGT TTT CAG        2310
   Arg Gly Ile Val Gln Gly Ala Val Asn Pro Phe Leu Thr Gly Phe Gln
               230                 235                 240
   GGA TTG GGA GTT GGG GCA ATT ACA GAC AGT GCG GTA AGC CCG GTA ACC        2358
   Gly Leu Gly Val Gly Ala Ile Thr Asp Ser Ala Val Ser Pro Val Thr
           245                 250                 255
   TAT GCG GCA GCA CGG AAA ACT TTA CAG GGT ATT CAC AAT TTA GGA AAT        2406
   Tyr Ala Ala Ala Arg Lys Thr Leu Gln Gly Ile His Asn Leu Gly Asn
       260                 265                 270
   TTA AGT CCG GAA GCA CAA CTT GCC GCC GCG AGC CTA TTA CAG GAC AGT        2454
   Leu Ser Pro Glu Ala Gln Leu Ala Ala Ala Ser Leu Leu Gln Asp Ser
   275                 280                 285                 290
   GCC TTT GCG GTA AAA GAC GGC ATC AAT TCC GCC AGA CAA TGG GCT GAT        2502
   Ala Phe Ala Val Lys Asp Gly Ile Asn Ser Ala Arg Gln Trp Ala Asp
                   295                 300                 305
   GCC CAT CCG AAT ATA ACA GCA ACA GCC CAA ACT GCC CTT GCC GTA GCA        2550
   Ala His Pro Asn Ile Thr Ala Thr Ala Gln Thr Ala Leu Ala Val Ala
               310                 315                 320
   GAG GCT GCA GGT ACG GTT TGG GGA GGT AAA AAA GTA GAA CTT AAC CCG        2598
   Glu Ala Ala Gly Thr Val Trp Gly Gly Lys Lys Val Glu Leu Asn Pro
           325                 330                 335
   ACC AAA TGG GAT TGG GTT AAA AAT ACC GGC TAT GAA AAA CCT GCT GCC        2646
   Thr Lys Trp Asp Trp Val Lys Asn Thr Gly Tyr Glu Lys Pro Ala Ala
       340                 345                 350
   CGA CCT ATG CAG ACT GTA GAC GGG GAA ATG GCC GGG AAA AAT AAG CCA        2694
   Arg Pro Met Gln Thr Val Asp Gly Glu Met Ala Gly Lys Asn Lys Pro
   355                 360                 365                 370
   CCG AAA CCA AGT ACG CAG CAA CAC TCT ACA CAC TCT GAT AAC AAT ATC      2742
   Pro Lys Pro Ser Thr Gln Gln His Ser Thr His Ser Asp Asn Asn Ile
                   375                 380                 385
   GGC TTA CCT GCC CCA TAT GTT AAA CCT GAT ACA TCT ATT TCT CCG ACA      2790
   Gly Leu Pro Ala Pro Tyr Val Lys Pro Asp Thr Ser Ile Ser Pro Thr
               390                 395                 400
   GGA ACA ATT CAA GAC CGC ATC AGA TGG ACA AAA TCC AAG TTT CCT ACT      2838
   Gly Thr Ile Gln Asp Arg Ile Arg Trp Thr Lys Ser Lys Phe Pro Thr
           405                 410                 415
   GAG AAA TCT TTA AAT GGA CAT TTC AAA GCT CAT GGA AAA GAA TTT GGC      2886
   Glu Lys Ser Leu Asn Gly His Phe Lys Ala His Gly Lys Glu Phe Gly
       420                 425                 430
   GAT ATA ACC ATT GAA GAC TAC CAA AAA ATG GCG TCT GAT TTG TTA TCA      2934
   Asp Ile Thr Ile Glu Asp Tyr Gln Lys Met Ala Ser Asp Leu Leu Ser
   435                 440                 445                 450
   AAA CAG ACA TCG GAC AAG ATA TTA GGT TAT CAG ACG GAA CAT AGA CGA      2982
   Lys Gln Thr Ser Asp Lys Ile Leu Gly Tyr Gln Thr Glu His Arg Arg
                   455                 460                 465
   GTG CGC TAT GAT ATC AAT AAC AAT ATC TAT GTT TTG GCC AAT CCA AAA      3030
   Val Arg Tyr Asp Ile Asn Asn Asn Ile Tyr Val Leu Ala Asn Pro Lys
               470                 475                 480
   ACA TTC AAA ATC AAA ACA ATG TTT AAA CCA AAC TTA GGA AAG GAG TAT      3078
   Thr Phe Lys Ile Lys Thr Met Phe Lys Pro Asn Leu Gly Lys Glu Tyr
           485                 490                 495
   TAT GAT GGA GAA TTC AAA AAA GAC ATG GGA AAT TGACGGAGAA ATATGGCTAC    3131
   Tyr Asp Gly Glu Phe Lys Lys Asp Met Gly Asn
       500                 505
   ATTGTCCTGT TTGCGGAACT GAAGTTATGG ACTATGATAT CTGTGACGTT TGTCAGTGGC    3191
   AAAATACAGG AGAAACTAAT ATAGATGGTG GTCCTAATGA AATGACACTT GCGGAGGCGA    3251
   AAGAAGCTTA CGCAAAAGGC TTACCAATCA GATAAA                              3287
(2) ANGABEN ZU SEQ ID NO: 2:
   (i) SEQUENZKENNZEICHEN:
      (A) LÄNGE: 1136 Basenpaare
      (B) ART: Nucleotid
      (C) STRANGFORM: Doppelstrang
      (D) TOPOLOGIE: linear
   (ii) ART DES MOLEKÜLS: Genom-DNA
   (iii) HYPOTHETISCH: NEIN
   (iv) ANTISENSE: NEIN
   (vi) URSPRÜNLICHE HERKUNFT:
      (A) ORGANISMUS: Neisseria gonorrheae
      (B) STAMM: MS11
   (ix) MERKMAL:
      (A) NAME/SCHLÜSSEL: CDS
      (B) LAGE:135..1094
   (xi) SEQUENZBESCHREIBUNG: SEQ ID NO: 2:
   AACAACAGTG AGATAAAAGT TCTTGTTCCA TTTTAAAACA ATTAGTAAAA TCGAGTTTAT      60
   CCTAGTTGTC CAGGACGGCC CCTAATTTAT TTACAATTTT GATACAATTT GTTTTTCATC     120
   AAAGGAGAAA ATCT ATG CGG GCA CGG CTG CTG ATA CCT ATT CTT TTT TCG       170
                   Met Arg Ala Arg Leu Leu Ile Pro Ile Leu Phe Ser
                   510                 515                 520
   GTT TTT ATT TTA TCC GCC TGC GGG ACA CTG ACA GGT ATT CCA TCG CAT       218
   Val Phe Ile Leu Ser Ala Cys Gly Thr Leu Thr Gly Ile Pro Ser His
               525                 530                 535
   GGC GGA GGC AAA CGC TTC GCG GTC GAA CAA GAA CTT GTG GCC GCT TCT       266
   Gly Gly Gly Lys Arg Phe Ala Val Glu Gln Glu Leu Val Ala Ala Ser
           540                 545                 550
   GCC AGA GCT GCC GTT AAA GAC ATG GAT TTA CAG GCA TTA CAC GGA CGA       314
   Ala Arg Ala Ala Val Lys Asp Met Asp Leu Gln Ala Leu His Gly Arg
       555                 560                 565
   AAA GTT GCA TTG TAC ATT GCA ACT ATG GGC GAC CAA GGT TCA GGC AGT       362
   Lys Val Ala Leu Tyr Ile Ala Thr Met Gly Asp Gln Gly Ser Gly Ser
   570                 575                 580                 585
   TTG ACA GGG GGT CGC TAC TCC ATT GAT GCA CTG ATT CGC GGC GAA TAC       410
   Leu Thr Gly Gly Arg Tyr Ser Ile Asp Ala Leu Ile Arg Gly Glu Tyr
                   590                 595                 600
   ATA AAC AGC CCT GCC GTC CGC ACC GAT TAC ACC TAT CCG CGT TAC GAA       458
   Ile Asn Ser Pro Ala Val Arg Thr Asp Tyr Thr Tyr Pro Arg Tyr Glu
               605                 610                 615
   ACC ACC GCT GAA ACA ACA TCA GGC GGT TTG ACG GGT TTA ACC ACT TCT       506
   Thr Thr Ala Glu Thr Thr Ser Gly Gly Leu Thr Gly Leu Thr Thr Ser
           620                 625                 630
   TTA TCT ACA CTT AAT GCC CCT GCA CTC TCG CGC ACC CAA TCA GAC GGT       554
   Leu Ser Thr Leu Asn Ala Pro Ala Leu Ser Arg Thr Gln Ser Asp Gly
       635                 640                 645
   AGC GGA AGT AGG AGC AGT CTG GGC TTA AAT ATT GGC GGG ATG GGG GAT       602
   Ser Gly Ser Arg Ser Ser Leu Gly Leu Asn Ile Gly Gly Met Gly Asp
   650                 655                 660                 665
   TAT CGA AAT GAA ACC TTG ACG ACC AAC CCG CGC GAC ACT GCC TTT CTT      650
   Tyr Arg Asn Glu Thr Leu Thr Thr Asn Pro Arg Asp Thr Ala Phe Leu
                   670                 675                 680
   TCC CAC TTG GTA CAG ACC GTA TTT TTC CTG CGC GGC ATA GAC GTT GTT      698
   Ser His Leu Val Gln Thr Val Phe Phe Leu Arg Gly Ile Asp Val Val
               685                 690                 695
   TCT CCT GCC AAT GCC GAT ACA GAT GTG TTT ATT AAC ATC GAC GTA TTC      746
   Ser Pro Ala Asn Ala Asp Thr Asp Val Phe Ile Asn Ile Asp Val Phe
           700                 705                 710
   GGA ACG ATA CGC AAC AGA ACC GAA ATG CAC CTA TAC AAT GCC GAA ACA      794
   Gly Thr Ile Arg Asn Arg Thr Glu Met His Leu Tyr Asn Ala Glu Thr
       715                 720                 725
   CTG AAA GCC CAA ACA AAA CTG GAA TAT TTC GCA GTA GAC AGA ACC AAT      842
   Leu Lys Ala Gln Thr Lys Leu Glu Tyr Phe Ala Val Asp Arg Thr Asn
   730                 735                 740                 745
   AAA AAA TTG CTC ATC AAA CCC AAA ACC AAT GCG TTT GAA GCT GCC TAT      890
   Lys Lys Leu Leu Ile Lys Pro Lys Thr Asn Ala Phe Glu Ala Ala Tyr
                   750                 755                 760
   AAA GAA AAT TAC GCA TTG TGG ATG GGG CCG TAT AAA GTA AGC AAA GGA      938
   Lys Glu Asn Tyr Ala Leu Trp Met Gly Pro Tyr Lys Val Ser Lys Gly
               765                 770                 775
   ATC AAA CCG ACG GAA GGA TTA ATG GTC GAT TTC TCC GAT ATC CGG CCA      986
   Ile Lys Pro Thr Glu Gly Leu Met Val Asp Phe Ser Asp Ile Arg Pro
           780                 785                 790
   TAC GGC AAT CAT ACG GGT AAC TCC GCC CCA TCC GTA GAG GCT GAT AAC     1034
   Tyr Gly Asn His Thr Gly Asn Ser Ala Pro Ser Val Glu Ala Asp Asn
       795                 800                 805
   AGT CAT GAG GGG TAT GGA TAC AGC GAT GAA GCA GTG CGA CAA CAT AGA     1082
   Ser His Glu Gly Tyr Gly Tyr Ser Asp Glu Ala Val Arg Gln His Arg
   810                 815                 820                 825
   CAA GGG CAA CCT TGATTCACAC TGCCATAACC GCTTGCTGCC AAGGAAAACA         1134
   Gln Gly Gln Pro
   AA                                                                  1136

## Beschreibung

Die vorliegende Erfindung betrifft Nucleinsäure-Moleküle aus Bakterien der Gattung Neisseria, die Proteine codieren, die die Adhäsion von Neisseria-Zellen an humane Zellen vermitteln. Ferner betrifft die vorliegende Erfindung die durch diese Nucleinsäure-Moleküle codierten Proteine und gegen diese gerichtete Antikörper. Die Erfindung betrifft weiterhin Arzneimittel, Impfstoffe und diagnostische Zusammensetzungen, die die beschriebenen Nucleinsäure-Moleküle, Proteine und/oder Antikörper enthalten.

Der Gattung Neisseria (gramnegative Kokken) gehören eine Reihe von Bakterienarten an, die als Saprophyten den oberen Respirationstrakt des Menschen besiedeln. Neben kommensalen Arten (e.g.: N. sicca) und opportunistisch pathogenen Arten (e.g.: N. lactamica) sind zwei Neisseria-Arten bekannt, welchen eindeutig humanpathogene Eigenschaften zuzuschreiben sind. Es handelt sich hierbei um die ausschließlich beim Menschen vorkommenden Arten N. gonorrhoeae, dem Erreger der gleichnamigen venerischen Erkrankung Gonorrhoe, sowie um N. meningitidis, dem Erreger der bakteriellen epidemischen Hirnhautentzündung. In beiden Fällen ist die Ätiologie, d.h. der kausale Zusammenhang zwischen Entstehung des Krankheitsbildes und Besiedelung durch Bakterien der genannten Arten hinlänglich gesichert.
Bei der durch N. meningitidis ("Meningokokkus") verursachten eitrigen Hirnhautentzündung (Meningitidis cerebrospinalis epidemica), die meist epidemisch auftritt, handelt es sich um eine systemische invasive Infektion der Hirn- und Rückenmarkshäute des Menschen. Gelegentlich werden zusätzlich hämorrhagische Exantheme am Rumpf, bzw. Begleiterkrankungen durch Herpes simplex beobachtet. Der Erreger kann in mehreren Serovars auftreten, die mittels Agglutinationsreaktionen mit Immunseren unterscheidbar sind. Die Hauptgruppen sind dabei bemerkenswert verschieden und kommen in räumlich und zeitlich unterschiedlicher Prävalenz vor. Meningokokken-Meningitidis trat bislang gehäuft alle 8 bis 12 Jahre auf mit einer Dauer der erhöhten Prävalenz von etwa 4 bis 6 Jahren. Während in den USA, sowohl bei der Zivilbevölkerung, als auch bei Militärpersonal, Serovar B Meningokokken 50% bis 55% der rezenten Erkrankungen auslösten, wurden in der ersten Hälfte des Jahrhunderts die meisten Epidemien in den USA durch Serovar A Menigokokken verursacht.
Das von N. gonorrhoeae verursachte Krankheitsbild ist meist eine lokal begrenzte Infektion der Schleimhäute vor allem des Urogenitaltrakts (Gonorrhoe), seltener der Konjunctiva (Conjunctivitis gonorrhoeae, Gonoblennorrhoe), die bei Neugeborenen perinatal, bei Erwachsenen meist einseitig über Schmierinfektion erworben wird. In sehr seltenen Fällen kommt es nach hämatogener Aussaat zu Bakteriämie und Sepsis, die Exantheme mit hämorrhagischen Pusteln und Folgeerkrankungen des rheumatischen Formenkreises, Arthritis gonorrhoica und/oder Endokarditis nach sich ziehen können.
Die Erkrankungen durch N. gonorrhoeae und N. meningitidis werden üblicherweise mit Antibiotika therapiert. In zunehmendem Maße treten jedoch Resistenzen gegen einzelne oder Gruppen der verwendeten Antibiotika auf, so daß die Verfügbarkeit der bislang nahezu ausschließlich praktizierten Therapiemethode eine ungünstige Prognose hat. Die Entwicklung alternativer Behandlungsmethoden, vorzugsweise präventiver Art, ist deshalb wünschenswert und dringlich.
Neisseria gonorrhoeae und N. meningitidis kommen ausschließlich beim Menschen vor und weisen in Anpassung an ihren einzigen Wirtsorganismus eine Vielzahl von Eigenschaften auf, die geeignet sind, die Abwehrmechanismen des Wirts wirkungslos zu machen. So ist bis heute kein Impfstoff verfügbar, der Gonorrhoe verhindert. Dies gilt in eingeschränkter Form auch für die Meningokokken-Meningitis. Obwohl die Erkrankung in jüngerer Zeit vorwiegend durch Bakterien desselben Serovars, Gruppe B, verursacht wird, ist ein wirksamer Impfstoff gegen Gruppe B Meningokokken bislang nicht existent. Impfstoffe gegen andere Serovars bieten nur partiellen Schutz und sind aus immunologischer Sicht nicht unproblematisch. Ursächlich für das Versagen der Immunabwehr ist unter anderem die antigene Variation auf der Erregerseite, die im Falle der pathogenen Neisserien besonders ausgeprägt ist. Eine Einschränkung des Freiraums der antigenen Variation ist jedoch dort denkbar, wo die sterische Aufrechterhaltung eines funktionellen Bereichs erforderlich ist, um die Interaktion mit weitgehend konservierten und konstanten Strukturen der Wirtsrezeptoren zu gewährleisten. Im Falle der Adhäsine, die zur Anheftung an die Wirtszelle dienen, ist diese Forderung in besonderem Maß zutreffend. Nur bei weitgehender Konstanthaltung des funktionellen an der physikalischen Interaktion beteiligten Bereichs ist die Wechselwirkung mit dem Rezeptor der Wirtszelle möglich. Dieser Bereich sollte damit antigener Variation weitgehend verschlossen sein und stellt damit einen geeigneten Ansatzpunkt für die Entwicklung neuer Therapiemethoden dar.

Bei einer Infektionserkrankung ist allgemein die stabile Anheftung der Erreger an Wirtsgewebe als Initialphase anzusehen. Durch Wechselwirkungen zwischen Strukturen der Zelloberflächen von Erreger und Wirtszelle wird eine mechanisch stabile Verbindung geschaffen, die ein Verweilen der Bakterien auf dem Gewebe des Wirts (Kolonisierung) und nachfolgende lokale Vermehrung der Erreger ermöglicht. Die Anheftung an die Wirtszelle ist in zwei Abschnitte gliederbar, wobei unterschiedliche Strukturen an der Wechselwirkung beteiligt sind.
Die erste Etappe der Adhäsion ist die Vermittlung eines Kontaktes zwischen Wirts- und Erregerzelle. Vielfach erfolgt die Kontaktaufnahme unter Beteiligung von Zellanhangs-Organellen, den sogenannten Pili. Diese auch Fimbrien oder Fibrillen genannten Zellorganellen sind wenige bis mehrere feine fadenförmige starre oder flexible Anhängsel der Bakterienzelle, deren Länge ein mehrfaches des Zelldurchmessers betragen kann. Bei der pilusvermittelten Adhäsion besteht deshalb noch kein Kontakt zwischen den Zellwänden von Erreger und Wirtszelle. Die Mehrzahl der bekannten Pili sind heteropolymere Strukturen, die aus verschiedenen Komponenten bestehen. Die meist in hoher Kopienzahl vorliegende Hauptuntereinheit erfüllt die Struktur- bzw. Gerüstfunktion, während die eigentliche Adhäsinfunktion von Nebenkomponenten getragen wird mit meist sehr niedriger Kopienzahl. In diesem Zusammenhang sei auch verwiesen auf DE 43 36 530 C, das rekombinante PilC-Proteine, deren Herstellung und Verwendung offenbart. Die Patentschrift betrifft ferner Antikörper gegen das PilC-Protein, Arzneimittel, Kits sowie zelluläre Rezeptoren für Typ 4-Pilus-tragende Bakterien und Analoga.
Bei einer weiteren Form der Adhärenz erfolgt die Anheftung von Erregern an die Wirtszellen ohne die Beteiligung von Pili (pilus independent adherence, pia). Hierbei kommt es nach räumlicher Annäherung zwischen Erreger- und Wirtszelle zur direkten Berührung der Zellwände. Diese Anlagerung und Stabilisierung des Zell-Zellkontaktes erfolgt unter Beteiligung von Adhäsinen, die in der Bakterienzellwand lokalisiert sind. Durch den direkten Zell-Zellkontakt kommt es schließlich zur Signalübertragung, welche die Erreger-induzierte Phagozytose einleitet und den Invasionsvorgang in die Zielzelle startet. Die pia Form der Adhärenz kann autonom die Anheftung der Erreger bewirken, beispielweise bei piluslosen Erregern. Sie kann aber auch als zweite Phase der Anheftung, d.h. als Folgereaktion nach pilusvermittelter Anheftung den zell-Zellkontakt stabilisieren. Die Adhäsine, die an der pilusunabhängigen Adhäsion beteiligt sind, können, aber müssen nicht andere Bindespezifitäten aufweisen, als die an der pilusabhängigen Adhäsion beteiligten Adhäsine.

Im Sinne der Erfindung werden nachfolgend die an der Anheftung beteiligten Strukturen der Bakterien als Adhäsine, die der Wirtszelle als Rezeptoren bezeichnet. Unterbleibt der Kontakt zwischen Adhäsin und Rezeptor, so bewirken "Abwehrmechanismen" des Wirts wie Flimmerbewegung der Epithelien, Mucusabsonderung, Massenströmungen von Körperflüssigkeiten u.a.m. die Elimination der Erreger. Die Entstehung einer Infektionserkrankung wird damit bereits im Vorfeld unterbunden. Eine Störung der Erregeranheftung durch Hemmung der Wechselwirkung zwischen Adhäsin und Rezeptor der Zielzelle stellt somit einen sehr wirkungsvollen Ansatz für die Prävention und Therapie von Infektionserkrankungen dar. Derartige therapeutisch wirksame Ansätze beinhalten die Bildung von Antikörpern, die spezifisch die Adhäsinfunktion blockieren, sei es durch aktive Immunisierung (Vakzinierung), oder durch Verabreichung bereits gebildeter Antikörper (passive Immunisierung). Eine Hemmung der Adhäsin-Rezeptor Bindung läßt sich ebenfalls mittels passiver Verabreichung sowohl von rezeptoranalogen, als auch von adhäsinanalogen Substanzen erzielen. Diese Stoffe binden kompetitiv an die jeweilige Partnerstruktur und blockieren diese damit für produktive Interaktionen. Solche Stoffe werden im Sinne der Erfindung als Inhibitoren bezeichnet.

Bislang durchgeführte Ansätze, unter Verwendung von Pilin, der Strukturfunktion tragenden Hauptkomponente des Pilus, eine breit wirksame Vakzine zu entwickeln, welche die Adhäsion von pathogenen Neisserien wirksam blockiert, schlugen fehl. Dies ist vermutlich darauf zurückzuführen, daß (i) Pilin selbst keine Adhäsinfunktion trägt und (ii) Pilin in besonders ausgeprägter Form intra- und interstammspezifische antigene Variation aufweist. Da beide Einschränkungen, wie oben dargelegt, für Adhäsine nicht gelten, sollte bei Verwendung eines Adhäsins als Vakzine diesem Ansatz eher Erfolg beschieden sein.

Der vorliegenden Erfindung liegt somit die Aufgabe zugrunde, Proteine und diese codierende DNA-Moleküle zur Verfügung zu stellen, die als Adhäsionsstrukturen bei Neisseria Spezies dienen oder zur Ausbildung solcher Strukturen beitragen.

Diese Aufgabe wird durch die Bereitstellung der in den Patentansprüchen bezeichneten Ausführungsformen gelöst.

Somit betrifft die vorliegende Erfindung Nucleinsäure-Moleküle, die die unter Seq ID No. 1 dargestellte Nucleotidsequenz oder Teile davon enthalten, wobei diese Nucleinsäure-Moleküle einen oder mehrere offene Leserahmen umfassen, die Proteine oder biologisch aktive Fragmente davon aus Bakterien der Gattung Neisseria codieren, die die Adhäsion von Neisseria-Zellen an humane Zellen vermitteln. Der Begriff "Leserahmen" wird hierbei synonym verwendet mit dem Begriff "codierende Region".
Gegenstand der Erfindung sind ebenso Nucleinsäure-Moleküle, die im Prinzip die in Seq ID No. 1 dargestellte Nucleotidsequenz aufweisen, bei denen jedoch die Nucleotidsequenzen der offenen Leserahmen aufgrund der Degeneration des genetischen Codes von denen der in Seq ID No. 1 angegebenen abweicht. Vorzugsweise weisen bei diesen Nucleinsäure-Molekülen die offenen Leserahmen Nucleotidsequenzen auf, die Proteine mit einer der unter Seq ID No. 1 angegebenen Aminosäuresequenzen codieren.
Gegenstand der Erfindung sind ferner Nucleinsäure-Moleküle, die mit den vorangehend beschriebenen Nucleinsäure-Molekülen hybridisieren und codierende Regionen umfassen, die Proteine codieren, die die Adhäsion von Neisseria-Zellen an humane Zellen vermitteln.
Im Rahmen der vorliegenden Erfindung wird der Begriff "Hybridisierung" verwendet, wie bei Sambrook et al. (Molecular Cloning, A Laboratory Manual; Cold Spring Harbor Laboratory Press (1989), 1.101 bis 1.104) beschrieben. Vorzugsweise wird darunter eine Hybridisierung unter stringenten Bedingungen verstanden. Darunter wird insbesondere eine Hybridisierung verstanden, bei der nach Waschen für 1 h mit 1 x SSC und 0,1 % SDS, vorzugsweise mit 0,2 x SSC und 0,1 % SDS, bei 55°C, vorzugsweise bei 62°C und besonders bevorzugt bei 68°C noch ein positives Hybridisierungssignal beobachtet werden kann.

In einer bevorzugten Ausführungsform stammt das erfindungsgemäße Nucleinsäure-Molekül aus einer pathogenen Neisserien Spezies, insbesondere aus Neisseria gonorrhoeae oder Neisseria meningitidis.

Der Begriff "Nucleinsäure-Molekül", wie hier erfindungsgemäß verwendet, bezieht sich auf eine polymere Form von Nucleotiden beliebiger Länge, entweder, als Ribonucleotide oder als Desoxyribonucleotide. Der Begriff bezieht sich lediglich auf die Primärstruktur des Moleküls. Sinngemäß umfaßt er folglich DNA- und RNA-Moleküle in Einzelstrang- oder Doppelstrangform. Bei der DNA kann es sich sowohl um cDNA als auch um genomische DNA handeln. Der Begriff umfaßt ferner die nicht modifizierte Form ebenso wie wissenschaftlich bekannte Arten der Modifikationen, z.B. Methylierung, "capping", Basensubstitution mit natürlichen oder synthetischen Analogen, Internucleotid-Modifikationen mit ungeladenen Verbindungen (z.B. Methyl-Phosphat, Phosphoamidat, Carbamat, Phosphotriester u.a.m.) oder mit geladenen Verbindungen (z.B. Phosphorothioat, Phosphorodithioat u.a.m.) oder mit Bindegliedern wie Proteinen und Peptiden (z.B. Nucleasen, Toxine, Antikörper, poly-L-Lysin u.a.m.). Der Begriff umfaßt auch Formen mit Intercalatoren (z.B. Acridin, Psoralen u.a.m.), Chelatoren (z.B. mit Metallen, radioaktiven Metallen oder oxidierenden Metallen, u.a.m.), solche mit alkylierenden Agentien und schließlich mit modifizierten Bindungen (z.B. alpha anomere Nucleinsäuren u.a.m.).

Die Erfindung betrifft ebenfalls Vektoren, die ein erfindungsgemäßes Nucleinsäure-Molekül enthalten. Bei dem Vektor kann es sich um einen beliebigen prokaryontischen oder eukaryontischen Vektor handeln. Beispiele für prokaryontische Vektoren sind chromosomale Vektoren, wie etwa Bakteriophagen (z.B. Bakteriophage Lambda, P1) und extrachromosomale Vektoren, wie etwa Plasmide, wobei zirkuläre Plasmidvektoren besonders bevorzugt sind. Geeignete prokaryontische Vektoren sind beispielsweise bei Sambrook et al. (s.o.), Kapitel 1 bis 4, beschrieben. Der erfindungsgemäße Vektor kann auch ein eukaryontischer Vektor sein, z.B. ein Hefevektor oder ein für höhere Zellen geeigneter Vektor (z.B. ein Plasmidvektor, ein viraler Vektor, ein Pflanzenvektor, u.a.m.). Beispiele für derartige Vektoren sind ebenfalls in Sambrook et al. (s.o., Kapitel 16) beschrieben. Ein Vektor, der ein erfindungsgemäßes Nucleinsäure-Molekül enthält, ist beispielsweise das Plasmid pES25 (enthalten im E. coli Stamm H 2560 (DSM 10257)). Der E. coli-Stamm H 2560 wurde am 18. September 1995 entsprechend den Anforderungen des Budapester Vertrages für die internationale Anerkennung der Hinterlegung von Mikroorganismen zum Zwecke der Patentierung bei der als internationale Hinterlegungsstelle anerkannten Deutschen Sammlung von Mikroorganismen (DSM) in Braunschweig, Bundesrepublik Deutschland unter der Hinterlegungsnummer DSM 10257 hinterlegt.

Ferner betrifft die Erfindung Wirtszellen, die einen oben beschriebenen Vektor enthalten oder mit einem oben beschriebenen Nucleinsäuremolekül genetisch manipuliert sind. Der Begriff "Wirtszelle" umfaßt im Rahmen dieser Erfindung sowohl prokaryontische als auch eukaryontische Wirtszellen. Prokaryontische Zellen sind bevorzugt, dabei besonders gramnegative prokaryontische Zellen, insbesondere E. coli-Zellen. Als eukaryontische Wirtszellen kommen beispielsweise Pilzzellen (z.B. Hefezellen), tierische oder pflanzliche Zellen in Frage.

Die unter Seq ID No. 1 dargestellte Nucleotidsequenz umfaßt drei offene Leserahmen. Diese stellen ein Operon dar, das eine funktionelle Einheit bildet. Die drei offenen Leserahmen, die als orfI , orfA und orfB bezeichnet werden, codieren drei Proteine, die im Rahmen dieser Erfindung als OrfI, OrfA und OrfB bezeichnet werden. Diese Sequenzen sind in Neisserien verantwortlich für die Expression eines Proteins, insbesondere des Proteins OrfA, das eine Funktion bei der Adhäsion von Neisseria-Zellen an humane Zellen besitzt. Die Proteine OrfI und OrfB besitzen offensichtlich Regulatorfunktion bzw. eine Funktion als Faktoren, die die Funktionalität von OrfA beeinflussen können.
Dieses Nucleinsäure-Molekül stellt somit eine Region des Neisserien-Genoms dar, die Proteine mit Adhäsin-Funktion von Neisserien codiert.

Ferner betrifft die vorliegende Erfindung auch Nucleinsäure-Moleküle, die ein Lipoprotein oder biologisch aktive Fragmente davon von Bakterien der Gattung Neisseria codieren, das die unter Seq ID No. 2 dargestellte Aminosäuresequenz aufweist. In einer bevorzugten Ausführungsform betrifft die Erfindung Nucleinsäure-Moleküle, die ein Protein codieren, das die Aminosäuresequenz von dem Aminosäurerest 19 bis zu dem Aminosäurerest 320 der in Seq ID No. 2 dargestellten Aminosäuresequenz aufweist. Bevorzugt besitzen derartige Nucleinsäure-Moleküle die unter Seq ID No. 2 dargestellt Nucleotidsequenz, insbesondere die Nucleotidsequenz von Nucleotid 189 bis Nucleotid 1095 der in Seq ID No. 2 dargestellten Sequenz.
Gegenstand der Erfindung sind ebenfalls Nucleinsäure-Moleküle, die ein Lipoprotein aus Bakterien der Gattung Neisseria codieren und deren Nucleotidsequenz von der der vorgehend beschriebenen Nucleinsäure-Moleküle aufgrund der Degeneration des genetischen Codes abweicht.
Ferner betrifft die vorliegende Erfindung Nucleinsäure-Moleküle, die ein Lipoprotein von Bakterien der Gattung Neisseria codieren und die mit einem der vorgehend beschriebenen Nucleinsäure-Moleküle hybridisieren (für die Definition des Begriffs "Hybridisierung" s.o.).

Gegenstand der Erfindung sind ebenfalls Fragmente, Derivate und allele Varianten der oben beschriebenen Nucleinsäure-Moleküle, die das oben genannte Lipoprotein codieren. Unter Fragmenten werden dabei Teile der Nucleinsäure-Moleküle verstanden, die lang genug sind, um das beschriebene Protein zu codieren. Der Ausdruck Derivat bedeutet in diesem Zusammenhang, daß die Nucleotidsequenzen dieser Moleküle sich von den Sequenzen der oben beschriebenen Nucleinsäure-Moleküle an einer oder mehreren Positionen unterscheiden und einen hohen Grad an Homologie zu dieser Nucleotidsequenz aufweisen. Homologie bedeutet dabei eine Sequenzidentität von mindestens 40 %, insbesondere eine Identität von mindestens 60 %, vorzugsweise über 80 % und besonders bevorzugt über 90 %. Die Abweichungen zu den oben beschriebenen Nucleinsäure-Molekülen können dabei durch Deletion, Substitution, Insertion oder Rekombination entstanden sein.
Homologie bedeutet ferner, daß funktionelle und/oder strukturelle Äquivalenz zwischen den betreffenden Nucleinsäure-Molekülen oder den durch sie codierten Proteinen, besteht. Bei den Nucleinsäure-Molekülen, die homolog zu den oben beschriebenen Nucleinsäure-Molekülen sind und Derivate dieser Nucleinsäure-Moleküle darstellen, handelt es sich in der Regel um Variationen dieser Moleküle, die Modifikationen darstellen, die dieselbe biologische Funktion ausüben. Es kann sich dabei sowohl um natürlicherweise auftretende Variationen handeln, beispielsweise um Sequenzen aus anderen Organismen, oder um Mutationen, wobei diese Mutationen auf natürliche Weise aufgetreten sein können oder durch gezielte Mutagenese eingeführt wurden. Ferner kann es sich bei den Variationen um synthetisch hergestellte Sequenzen handeln.
Bei den allelischen Varianten kann es sich sowohl um natürlich auftretende Varianten handeln, als auch um synthetisch hergestellte oder durch rekombinante DNA-Techniken erzeugte Varianten.
Die von den verschiedenen Varianten der erfindungsgemäßen Nucleinsäure-Moleküle codierten Proteine weisen bestimmte gemeinsame Charakteristika auf. Dazu können z.B. Enzymaktivität, Molekulargewicht, immunologische Reaktivität, Konformation etc. gehören, sowie physikalische Eigenschaften wie z.B. das Laufverhalten in Gelelektrophoresen, chromatographisches Verhalten, Sedimentationskoeffizienten, Löslichkeit, spektroskopische Eigenschaften, Stabilität, pH-Optimum, Temperatur-Optimum etc.

Vorzugsweise weisen die von den erfindungsgemäßen Nucleinsäure-Molekülen codierten Proteine eine Homologie von 80 %, besonders bevorzugt von über 90 % zu der unter Seq ID No. 2 dargestellten Nucleotidsequenz auf.

Die vorstehend beschriebenen Nucleinsäure-Moleküle codieren ein Lipoprotein aus Bakterien der Gattung Neisseria. Dieses Protein wird im Rahmen der vorliegenden Erfindung als OrfA bezeichnet. Dieses Protein ist experimentellen Daten zufolge auf der Zelloberfläche von Neisserien lokalisiert, insbesondere auf der äußeren Membran. Das Protein weist vorzugsweise ein Molekulargewicht von ca. 36 kD auf, wenn es im T7-Expressionssystem analysiert wird. Ferner besitzt dieses Protein eine biologische Aktivität, die die Adhäsion von Neisseria-Zellen an humane Zellen vermittelt. Dies erfolgt insbesondere dadurch, daß dieses Protein mit dem als PilC bekannten Protein aus Neisseria einen Komplex bildet. Die Adhäsion findet dabei bevorzugt auf humanen Epithelzellen statt.

Ferner betrifft die Erfindung Vektoren, die die vorstehend beschriebenen Nucleinsäure-Moleküle enthalten. Beispiele für derartige Vektoren wurden bereits oben beschrieben.

In einer bevorzugten Ausführungsform sind in derartigen Vektoren die erfindungsgemäßen DNA-Moleküle mit regulatorischen DNA-Elementen verknüpft, die die Expression des Proteins in pro- oder eukaryontischen Zellen ermöglichen. Darunter werden im Rahmen dieser Erfindung beispielsweise Promotoren, Operatoren, Enhancer u.ä. verstanden.

Ferner betrifft die Erfindung Wirtszellen, die vorstehend beschriebene erfindungsgemäße Vektoren enthalten oder mit den vorstehend beschriebenen Nucleinsäuremolekülen genetisch manipuliert sind. Genetisch manipuliert bedeutet dabei, daß in die Wirtszelle oder in eine Vorgängerzelle ein derartiges Molekül mittels (gen)technischer Methoden eingeführt wurde. Als Wirtszellen kommen wiederum die oben beschriebenen in Frage.

Ebenso betrifft die Erfindung Verfahren zur Herstellung des beschriebenen Lipoproteins oder eines biologisch aktiven Fragmentes davon, bei dem die oben beschriebenen Wirtszellen unter Bedingungen kultiviert werden, die die Expression des Proteins erlauben, und das Protein aus den Zellen und/oder dem Kulturüberstand isoliert wird.

Die Erfindung betrifft ebenfalls die Proteine, die von einem der vorstehend beschriebenen Nucleinsäure-Moleküle codiert werden, sowie biologisch aktive Fragmente davon, sowie Proteine, die durch das vorangehend beschriebene Verfahren erhältlich sind. Die Beschreibung verweist auch auf Proteine, die mit den beschriebenen Proteinen immunologisch kreuzreagierende Aminosäuresequenzen aufweisen. Der Begriff "Protein" umfaßt im Rahmen der vorliegenden Erfindung auch natürlich vorkommende Varianten oder Modifikationen oder Fragmente, bzw. synthetisch hergestellte Modifikationen, Varianten oder Fragmente mit entsprechender biologischer Aktivität. Abgeleitete oder rekombinante Proteine müssen nicht notwendigerweise auf biologischem Weg von der Nucleotidsequenz übersetzt werden. Sie können auf beliebige Art hergestellt werden, einschließlich chemischer Synthese, in vitro Synthese mittels eines Expressionssystems oder durch Isolierung aus Organismen. Erfindungsgemäße Proteine können auch eine oder mehrere Aminosäureanaloge oder nicht natürlich vorkommende Aminosäuren enthalten. Ebenso können Modifikationen (z.B. Glykosylierung, u.a.m.) oder Markierung (z.B. Biotinylierung, u.a.m.) nach wissenschaftlichem Kenntnisstand enthalten sein.
Die Fragmente haben vorzugsweise eine Mindestlänge von 3 bis 5 Aminosäuren, besonders bevorzugt von 8 bis 10 Aminosäuren und insbesondere von 11 bis 15 Aminosäuren. Das vorstehende trifft auch auf die weiter unten beschriebenen erfindungsgemäßen Proteine zu.

Das erfindungsgemäße Lipoprotein OrfA läßt sich beispielsweise durch ein Verfahren reinigen, das auf der Wechselwirkung dieses Proteins mit dem PilC-Protein aus Neisseria gonorrhoeae beruht. Die Reinigung erfolgt dabei vorzugsweise aus Homogenaten von Zellen, die dieses Protein exprimieren, mittels Chromatographie-Matrizes, die immobilisiertes PilC-Protein enthalten. Das Protein läßt sich dann unter Ausnutzung der Affinität zu PilC selektiv eluieren und in wesentlich reiner Form darstellen.

Die erfindungsgemäßen Proteine oder Fragmente davon können als Immunogene zur Herstellung von Antikörpern verwendet werden. Somit betrifft die vorliegende Erfindung auch Antikörper, die gegen ein erfindungsgemäßes Protein oder ein Fragment davon gerichtet sind. Die Antikörper können sowohl polyclonal als auch monoclonal sein. Verfahren zur Herstellung derartiger Antikörper sind dem Fachmann geläufig.

In einer bevorzugten Ausführungsform sind derartige Antikörper gegen Epitope des erfindungsgemäßen Proteins oder Fragmente davon gerichtet, die für die Adhärenz und für die Interaktion mit PilC wesentlich sind.

Die Erzeugung der erfindungsgemäßen Antikörper kann beispielsweise auch dadurch erfolgen, daß die oben beschriebenen erfindungsgemäßen Nucleinsäuresequenzen durch in vivo Transfektion in Wirte eingebracht werden. Dadurch kommt es in dem Wirt zur Expression des Proteins oder eines Fragmentes davon und zur Induktion von dagegen gerichteten Antikörpern (Nucleinsäure-Vakzinierung). Dies gilt auch für die weiter unten beschriebenen Antikörper.

Die Beschreibung verweist ferner auf Nucleinsäure-Moleküle, die mindestens 12 Nucleotide lang sind und die spezifisch mit einem vorangehend beschriebenen Nucleinsäure-Molekül hybridisieren. Derartige Nucleinsäure-Moleküle können eine Mindestlänge von 15 Nucleotiden, oder von 20 Nucleotiden aufweisen. Solche Moleküle eignen sich beispielsweise als Primer für in vitro Amplifikation, z.B. mittels Polymerase-Kettenreaktion (PCR), oder zur Diagnose, d.h. zum spezifischen Nachweis der erfindungsgemäßen Nucleinsäure-Moleküle in Proben.

Ferner betrifft die Erfindung Arzneimittel, die ein vorstehend beschriebenes erfindungsgemäßes Nucleinsäure-Molekül, Protein, ein biologisch aktives Fragment davon und/oder einen oben beschriebenen erfindungsgemäßen Antikörper enthalten. Im Rahmen der vorliegenden Erfindung können derartige Arzneimittel gegebenenfalls die üblichen pharmazeutischen Hilfs-, Verdünnungs-, Zusatz- und/oder Trägerstoffe enthalten. Ebenso betrifft die Erfindung Impfstoffe, die vorstehend beschriebene Nucleinsäure-Moleküle, Proteine, biologisch aktive Fragmente davon und/oder Antikörper enthalten.

In einem weiteren Aspekt betrifft die vorliegende Erfindung diagnostische Zusammensetzungen, die die vorstehend beschriebenen erfindungsgemäßen Nucleinsäure-Moleküle, Proteine, biologisch aktive Fragmente davon und/oder Antikörper enthalten.

Ein weiterer Aspekt der Beschreibung sind Rezeptoren und Stoffe mit Rezeptorfunktion, die als Liganden mit dem erfindungsgemäßen Adhäsin, dem OrfA-PilC-Komplex interagieren. Solche Stoffe sind aufgrund ihrer Wechselwirkung mit dem OrfA-PilC-Komplex als kompetitive Hemmstoffe der Adhärenzfunktion identifizierbar. Dabei kann es sich um Oberflächenkomponenten von humanen Zellen, in einer besonders bevorzugten Form um Oberflächenkomponenten von humanen Epithelzellen oder um chemische Substanzen beliebiger Herkunft handeln.

Die Beschreibung verweist ferner auf Inhibitoren, welche die Wechselwirkung zwischen dem OrfA-PilC-Adhäsin-Komplex und seinen Rezeptoren beeinflussen. Dies Können Substanzen, sein, welche die Interaktion zwischen dem OrfA-PilC Adhäsin und seinem zellulären Rezeptor beeinflussen und damit die Adhärenz stören.

Die im Rahmen der vorliegenden Erfindung beschriebenen pharmazeutischen Zusammensetzungen können einerseits zur Identifizierung und Charakterisierung einer nicht bekannten Bakterienprobe als pathogene Neisseria spc., andererseits auch zur Diagnostik einer Neisseria Infektion verwendet werden. Auf Polynucleotidebene erfolgt die Verwendung vorzugsweise über Hybridisierungssonden, welche erfindungsgemäße Nucleotidsequenzen umfassen, die für einen der orf-Genbereiche spezifisch sind, oder durch die Verwendung erfindungsgemäßer Nucleotidsequenzen aus dem orfA-Genbereich als Primer für die PCR-Amplifikation des nachzuweisenden, für pathogene Neisserien spezifischen genomischen DNA Abschnitts.
Auf Polypeptidebene erfolgt die Diagnostik vorzugsweise mit Hilfe der erfindungsgemäßen Antikörper, bzw. bei Antikörpersuchtests mit Hilfe der erfindungsgemäßen immunogenen Proteine oder Fragmenten davon.
Rezeptoren, rezeptoranaloge Substanzen und Inhibitoren der Wechselwirkung zwischen erfindungsgemäßem OrfA und zugehörigen Rezeptoren der Wirtszellen Könnten Anwendung finden als Therapeutikum bei bestehender Infektion im Frühstadium, oder bei Verdacht auf Infektion. Durch nachhaltige Hemmung der Adhärenz kann die Anheftung der Erreger an Wirtsepithelien verhindert werden, so daß vermittels der üblichen Abwehrmechanismen wie Flimmerbewegung der Epithelien, Mucusabsonderung, Massenströmungen von Körperflüssigkeiten u.a.m. die Erreger eliminiert werden.
Schließlich können die erfindungsgemäßen pharmazeutischen Zubereitungen auch zur Prävention oder Bekämpfung von Neisseria Infektionen verwendet werden. Vorzugsweise werden für präventive Anwendungen die erfindungsgemäßen Proteine oder Teile davon zur Herstellung eines Impfstoffs für aktive Immunisierung, oder erfindungsgemäße Antikörper zur Herstellung eines als Therapeutikum einsetzbaren passiven Impfstoffs verwendet. Die vorstehend erläuterten Verwendungsmöglichkeiten treffen auch auf die weiter unten beschriebenen Arzneimittel und diagnostischen Zusammensetzungen zu.

Erläuterungen zu den Figuren und Sequenzprotokollen:
- Figur 1: zeigt schematisch den Aufbau des Plasmids pES25.

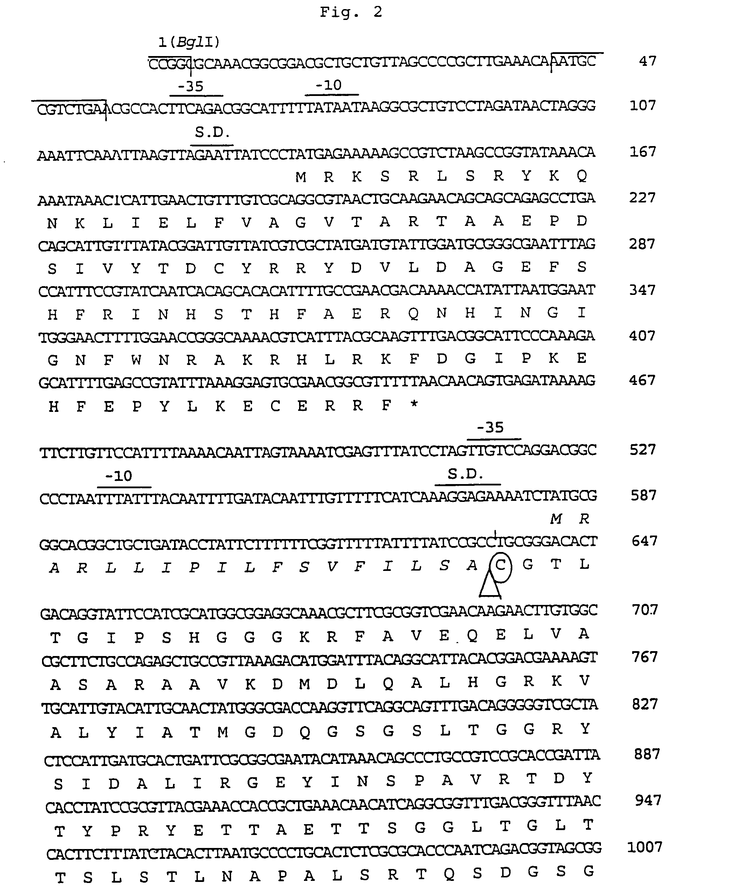

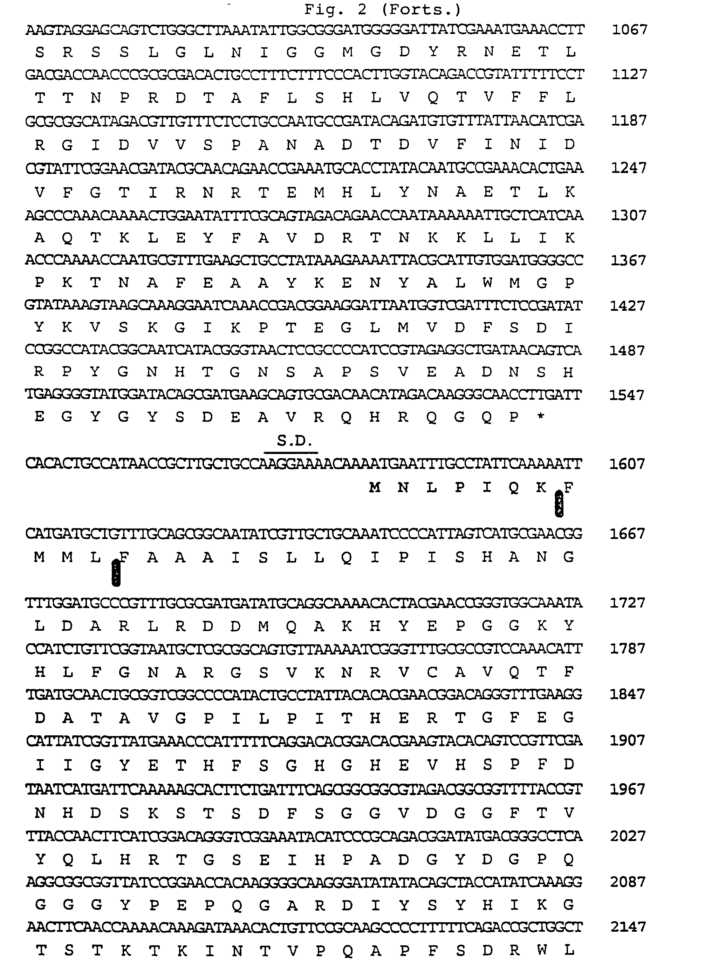

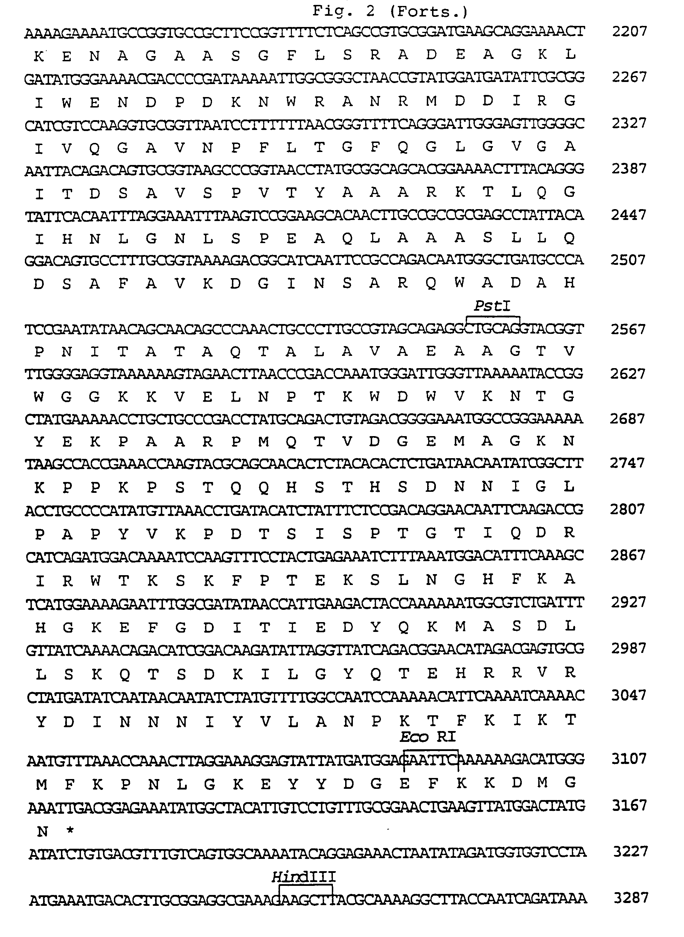

- Figur 2: zeigt die Nucleotidsequenz (SEQ ID No. 1) des orf Genbereichs, beginnend mit Position 1 an der modifizierten BglI-Schnittstelle und endend mit Position 3260, des letzten Nucleotids der HindIII-Schnittstelle. Restriktionsschnittstellen, Ribosomenbindestellen (Shine-Dalgarno-Sequenzen) und Promotor-Sequenzen (-35- und -10-Regionen) sind gekennzeichnet.
SEQ ID No. 1 zeigt ferner die Aminosäuresequenzen der vom *orf*-Genbereich codierten Proteine OrfI , OrfA und OrfB. Die Aminosäuren der Lipoprotein-Signalsequenz von OrfA sind kursiv geschrieben, die Schnittstelle der Lipoprotein-Signalpeptidase II ist mit einer Pfeilspitze gekennzeichnet. Die Aminosäure Cystein, die den Aminoterminus des prozessierten OrfA Lipoproteins darstellt und zu Glyzerylcystein und mit Fettsäure modifiziert wird, ist eingekreist. Die ersten sieben Aminosäuren von OrfB, die Ähnlichkeit mit einer TypIV-Pilin-Signalsequenz aufweisen, sind fettgedruckt. Die Markierungen zwischen Aminosäuren 7 und 8, bzw. 11 und 12 kennzeichnen potentielle Schnittstellen analog zur Prozessierung des TypIV-Pilins.
- Seq ID No. 2: zeigt die Nucleotidsequenz des Genbereichs, der OrfA codiert, sowie flankierende Sequenzen. Die Aminosäuresequenz von OrfA ist ebenfalls angegeben.

Die Beispiele erläutern die Erfindung.

### Beispiel 1

### Methode zur Isolierung des Lipoprotein-Adhäsins OrfA:

Für die Identifizierung des erfindungsgemäßen neuen Adhäsins von Neisseria gonorrhoeae erwies sich eine Beobachtung bei der chromatographischen Reinigung des PilC Proteins als entscheidend. Hierbei wurde rekombinantes PilC Protein verwendet, das zur Erleichterung der chromatographischen Aufreinigung durch einen Oligo-Histidin Bereich mit sechs Histidin-resten (His₆-tag) erweitert wurde (Rudel et al., Nature 373, 357-359, 1995). Die Erweiterung des Proteins um das Histidinhexapeptid ermöglicht die selektive Bindung an eine Nickel-Nitrilotriacetat-Agarosematrix (Ni-NTA-Matrix). Nach Extraktion der Zellwändfraktion, hergestellt aus Kulturen eines piluslosen PilC-Überproduzentenstamms N560 (Rudel et al., s.o.) von Neisseria gonorrhoeae, wurde folglich das Extrakt auf eine Ni-NTA-Chromatographie Matrix aufgetragen. Üblicherweise wurde bei dem zur Reinigung von rekombinantem PilC entwickelten Verfahren unspezifisch gebundenes Material durch ausgiebiges Waschen mit einem imidazolhaltigen Puffer entfernt. Es konnte jedoch in der ersten Elutionsfraktion zusammen mit PilC ein 36 kD Protein (OrfA) in etwa äquimolarem Verhältnis nachgewiesen werden.

Zur Präparation der PilC-OrfA Proteinfraktion wurde der Stamm N560 von Neisseria gonorrhoeae auf 30 GC-Agar-Platten ausgestrichen und 20 h bei 37°C in 5% CO₂ inkubiert. Das GC-Agar-Medium (GC Agar Base, Becton Dickinson, Heidelberg) enthielt die üblichen für das Wachstum von Neisseria gonorrhoeae erforderlichen Zusatzfaktoren (0,1 mg Vitamin B12, 10 mg Adenin, 0,3 mg Guanin, 100 mg Glutamin, 1 mg Cocarboxylase, 0,3 mg Thiamin, 259 mg L-Cystein, 11 mg L-Cystin, 1,5 mg Arginin, 5 mg Uracil, 0,2 mg Fe(NO₃,)₃, 2,5 mg Diphosphopyridinnucleotid, 0,13 mg p-Aminobenzoesäure und 1 g Dextrose pro 1 Liter Medium), die als Sterilfiltrat dem GC Basismedium nach dessen Hitzesterilisation zugesetzt wurden. Außerdem enthielt das so komplettierte GC-Agar-Medium 5 µg/ml Tetracyclin, und 100 µM IPTG. Die Bakterienrasen wurden mit Wattetupfern abgenommen, in 30 ml Waschpuffer (Tris-HCl pH 8,0 mit 0,15 M NaCl) transferiert und bei 4.000 UpM, 4°C für 15 min zentrifugiert (Du Pont Sorvall Zentrifuge RC-5B, Rotor SS-34). Das Zellsediment wurde erneut in 30 ml Waschpuffer resuspendiert und die Bakterien nach Zugabe von Lysozym und 5 mM EDTA· Na₂ durch Ultraschall-Homogenisation aufgeschlossen. Intakte Bakterien wurden durch Zentrifugation bei 5.000 UpM, 4°C für 15 min abgetrennt. Die Zellhüllen der lysierten Bakterien wurden durch Zentrifugation des Überstands bei 20.000 UpM, 4°C für 60 min sedimentiert und anschließend in 10 ml Waschpuffer aufgenommen, der zusätzlich 10% Glyzerin, 10 mM MgCl₂ und 2% Triton X-100 enthielt. Nach 45 min Inkubation bei 37°C wurde erneut zentrifugiert (20.000 UpM, 4°C für 60 min) und das Membransediment in 10 ml Waschpuffer mit 10 % Glyzerin, 10 mM MgCl₂, und 2% LDAO (N,N-Dimethyldodecylamin-N-oxid) suspendiert und bei 37°C für 60 min inkubiert. Nach erneuter Zentrifugation (20.000 UpM, 4°C für 60 min) wurde der Überstand, der den biologisch aktiven PilC-OrfA Komplex enthielt zur weiteren Reinigung einer Nickel-Chelat-Affinitätschromatographie unterzogen. Hierfür wurde Ni-NTA-Gelmatrix (300 ml Bettvolumen) mit 5 Bettvolumina Aqua bidest. gewaschen und danach 10 ml des Überstandes aufgetragen. Unspezifisch gebundene Proteine wurden durch Elution mit 5 Säulenvolumina 50 mM Imidazol in PBS Puffer pH 8,0 enfernt. Nach erneutem Waschen der Säule mit 5 bis 10 Bettvolumina 20 mM Natriumphosphatpuffer pH 7,5 mit 0,15 M NaCl (PBS Puffer) wurde der biologisch aktive PilC-OrfA Komplex mit Citrat/Phosphat-Puffer (10 mM Citronensäure, 1 M Natriumphosphat, pH 3,5, 10% Glyzerin, 0,15 M NaCl) in der ersten Elutionsfraktion eluiert und sofort mit einer 1 M Na₂HPO₄-Lösung neutralisiert. Das Eluat, das PilC und OrfA enthielt wurde in flüssigem Stickstoff eingefroren und bei -70°C gelagert.

### Beispiel 2

### Isolierung der Polynucleotidsequenz, die den orf-Genbereich trägt

Um das 36 kD OrfA Protein näher zu charakterisieren wurden Mäuse mit der PilC-36 kD Proteinfraktion immunisiert. Das 36 kD Protein erwies sich dabei als sehr immunogen. Mit Hilfe der so gewonnenen Antikörper wurde eine pBA Plasmid-Genbank des Neisseria gonorrhoeae MS11 Genoms in E. coli GC1 auf Antigen-Präsenz gescreent. Mehrere Clone mit positiver Reaktion wurden isoliert und Clon H1967 zur weiteren Charakterisierung ausgewählt.

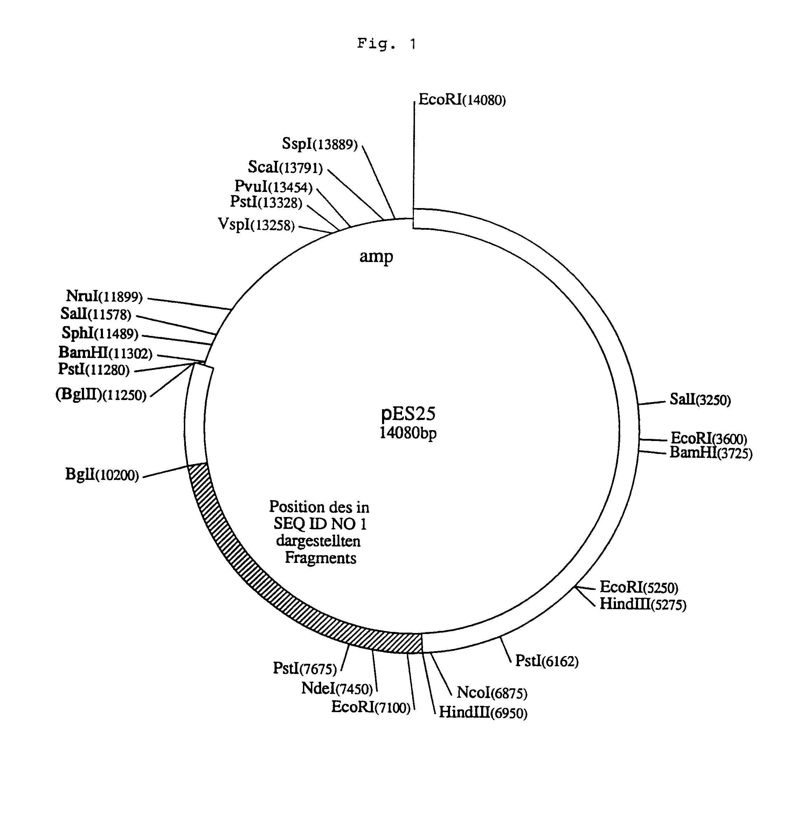

Das Genbankplasmid pES25 (Figur 1) von Clon H1967 enthielt ein genomisches Fragment von etwa 11 kb, cloniert im Vektor pBA. Restriktionsfragmente des Gesamtbereichs wurden subcloniert in pUC- bzw. pBluescript KS (+) -Vektoren. Mit Hilfe von Expression der abgeleiteten Plasmide in Minizellen und Immunoblot Analysen wurden Subclone ausgewählt, die das 36 kD Protein produzierten.

Diese wurden für die Sequenzierung herangezogen. Die Sequenzbestimmung erfolgte durch direkte Sequenzierung von Restriktionsfragmenten, durch Sequenzierung von kontinuierlich verkürzten ExoIII-Nucleasefragmenten des BglI-PstI Fragments (Pos. 1 bis 2560 von Seq ID No. 1), sowie durch Sequenzierung von PCR amplifizierten Fragmenten.

Die in SEQ ID No.1 dargestellte Region von der BglI-Schnittstelle (Pos. 1) bis zur HindIII-Schnittstelle (Pos. 3260) wies drei offene Leseraster mit hoher Codierungswahrscheinlichkeit auf, wobei jedes Leseraster mit dem Startcodon ATG beginnt, in passendem Abstand eine dem Startcodon vorhergehende Ribosomenbindestelle aufweist (S.D.-Sequenz) und mit einem Stopcodon endet.

Die drei Leserahmen weisen dieselbe Orientierung auf. Der erste offene Leserahmen beginnt bei Position 136 der in SEQ ID No. 1 dargestellten Sequenz und endet bei Position 450 mit dem Stopcodon TAA. Das codierte Protein wurde OrfI genannt und zeigt im T7-Expressionssystem ein apparentes Molekulargewicht von 18 kD. Durch Sequenzvergleich konnten in der EMBL Genbank (Release 43.0 aus 6/95) und in der SwissProt Datenbank (Release 31.0 aus 3/95) weder auf Nucleotidsequenzebene, noch auf Aminosäurensequenzebene signifikante Homologe identifiziert werden.

Der zweite offene Leserahmen beginnt bei Position 583 und endet bei Position 1545 mit dem Stopcodon TGA. Er codiert für das OrfA Protein, das im T7-Expressionssystem ein apparentes Molekulargewicht von 36 kD zeigt. Auch zu dieser Sequenz konnte mittels Datenbanksuche keine signifikanten Homologe aufgefunden werden. Die Sequenzanalyse mittels des Protein Analyse-Programms "Motifs" (GCG Genetics Computer Group, Inc., Madison, Wisconsin, U.S.A.) ergab jedoch eine vollständige Homologie des N-Terminus von OrfA mit Lipoprotein-spezifischen Signalsequenzen (Pos. 583 bis 636). Die Charakterisierung von OrfA als Lipoprotein konnte auf experimentellem Weg bestätigt werden (Vide infra).

Der dritte offene Leserahmen beginnt bei Position 1585 und endet bei Position 3114 mit dem Stopcodon TGA. Das hierdurch codierte Protein, OrfB, weist im T7-Expressionssystem ein apparentes Molekulargewicht von 57 kD. Auch zu diesem Leserahmen konnten durch Datenbanksuche kein Homolog identifiziert werden.

An strukturellen Besonderheiten weist der Aminoterminus der OrfB-Sequenz eine Signalsequenz auf, die Ähnlichkeiten zur Typ IV-Präpilin-Signalsequenz zeigt. An Positionen 8 und 12 der Aminosäurensequenz befindet sich Phenylalanin, so daß zudem zwei potentielle Schnittstellen für die Typ IV-Pilin Signalpeptidase existieren. Es läßt sich hieraus ableiten, daß es sich bei OrfB vermutlich um ein sekretiertes Protein handelt.

Die im T7-Expressionssystem ermittelten Molekulargewichte aller drei Genprodukte stimmen mit den theoretischen aus der Sequenz errechneten Werten überein. Die gelelelektrophoretische Trennung der Expressionsprodukte zeigte, daß die OrfB-Bande in allen Fällen deutlich schwächer ausgeprägt war, als die OrfA-Bande. Dies weist auf eine schwächere Expression von OrfB hin.

Zwei Bereiche mit Sequenzhomologie zu Promotorbereichen wurden identifiziert. Der eine liegt vor dem orfI Gen, der zweite vor dem orfA Gen, jeweils in passendem Abstand (SEQ ID No. 1). Es ist somit anzunehmen, daß orfA und orfB eine Transkriptionseinheit bilden.

Die Analyse des Neisseria gonorrhoeae MS11 Genoms nach ClaI und MluI Verdau zeigte in Southern Hybridisierung mit Plasmid pES-8 als Probe ein komplexes Bandenmuster. Dies weist auf die Existenz mehrerer Kopien des orf-Genbereichs, vermutlich drei Kopien, im Genom von Neisseria gonorrhoeae MS11 hin. Ob alle diese Loci exprimiert sind, ob sie antigener Variation unterliegen wie beispielsweise die Neisseria-Gene pilS und opa, und ob die flankierenden Regionen des orf-Genbereichs an den Sequenzwiederholungen beteiligt sind, ist gegenwärtig nicht bekannt.

### Beispiel 3

### Charakterisierung der Lokalisierung von OrfA und OrfB an der Zelloberfläche

Um die aus der perfekten Strukturhomologie des Aminoterminus von *orfA* mit Lipoproteinsignalsequenzen ableitbare Lipoproteinnatur von OrfA experimentell zu beweisen, wurden sowohl N. gonorrhoeae, als auch mit dem orf-Genbereich transformierte E. coli Rekombinanten mit [³H]Palmitat markiert. Die Ergebnisse der Markierung zeigen, daß in allen Fällen, sowohl bei N. gonorrhoeae, als auch bei den E. coli Rekombinanten Lipoproteine im entsprechenden Molekulargewichtsbereich identifiziert wurden. Während bei N. gonorrhoeae mehrere Proteine markiert wurden und eine präzise Zuordnung der markierten Banden wegen der Nichtverfügbarkeit einer *orfA*⁻-Mutante nicht durchführbar war, zeigen die orfA-Rekombinanten von E. coli im Vergleich zum Kontrollstamm ganz eindeutig nur eine einzige zusätzlich Bande mit dem Molekulargewicht von OrfA. Ein zusätzlich getestetes OrfA-Fusionsprotein, das am Carboxyterminus durch eine Fusion um 3 kD vergrößert wurde, wies ebenfalls eine [³H]Palmitat-Markierung auf und migrierte an eine Position, die exakt dem erwarteten fusionsbedingt höheren Molekulargewicht entsprach.

Bei Behandlung präparierter Zellhüllen mit Detergentien zeigte OrfA eine Löslichkeit, wie sie für Proteine der äußeren Membran typisch ist. Durch Auftrennung der Zellhülle mittels Dichtegradienten-Zentrifugation konnte die Lokalisierung von OrfA in der äußeren Membran von N. gonorrhoeae anhand von Leitproteinen bestätigt werden. Auch bei den orf-Rekombinanten von E. coli wurde OrfA mittels der genannten Technik als Proteinkomponente der äußeren Membran nachgewiesen.

Die Zugänglichkeit an der Zelloberfläche wurde mittels Immunfluoreszenz-Test sowohl für OrfA, als auch OrfB gezeigt. Eine pilC Defektmutante von Neisseria gonorrhoeae, deren beide pilC Gene ausgeschaltet sind, wird durch das PilC-OrfA Antiserum ebenso markiert, wie rekombinante E. coli Stämme, die den orf-Genbereich tragen. Der nichttransformierte Kontrollstamm verhielt sich, wie zu erwarten, negativ. Eine positive Reaktion im Immunfluoreszenztest von N. gonorrhoeae und orf-rekombinanten E. coli Stämmen ließ sich ebenfalls mit OrfA- und OrfB- spezifischen Antiseren erzeugen, wobei zur Herstellung dieser Antiseren gereinigte Fusionsproteine von entweder OrfA oder OrfB verwendet wurden. Wurde dagegen Antiserum verwendet, das gegen ein OrfI Fusionsprotein gerichtet war, so verlief der Immunfluoreszenztest mit N. gonorrhoeae negativ. Daraus läßt sich ableiten, daß OrfA und OrfB an der Zelloberfläche lokalisiert und von außen zugänglich sind, während für OrfI eine intrazelluläre Lokalisation wahrscheinlich ist.

Die Oberflächenlokalisierung von OrfA und OrfB ließ sich nur in rekombinanten E. coli Stämmen nachweisen, die den gesamten orf-Bereich trugen.

### Beispiel 4

### Adhäsin-Eigenschaft des OrfA-PilC Komplexes

Wie oben erwähnt ließ sich OrfA durch seine Affinität zu PilC über Chromatographie an einer Ni-NTA-Chelat-Matrix in reiner Form darstellen. Da für PilC die Funktion als pilusassoziiertes Adhäsin nachgewiesen und ebenfalls die direkte Bindung von PilC an humane ME-180 Zellen bekannt war, lag es nahe, die Adhärenzeigenschaft des PilC-OrfA-Komplexes zu untersuchen. Die Experimente hierzu wurden mit dem E. coli Stamm HB101 (E141) durchgeführt, weil dieser keine Mannose-spezifischen TypI Pili besitzt und so gut wie keine Bindung an humane ME-180 und Chang Epithelzellen zeigt. Nach Transformation von HB101 mit dem Plasmid pES25, konnte weder eine Adhärenz an ME-180 noch an Chang-Zellen vermittelt werden. Wurden dieselben Rekombinanten jedoch mit gereinigtem PilC Protein vorinkubiert, so konnte eine starke Adhärenz an Chang Epithelzellen, nicht aber an ME-180 Zellen induziert werden (Tabelle I).

**Tabelle I**

| **OrfA-abhängige Modulation der PilC-vermittelten Adhäsinfunktion** | | |
|---|---|---|
| | Adhärenz an humane Epithelzellen | |
| | ME180-Zellen | Chang-Zellen |
| N. gonorrhoeae, Orf+ PilC+, Pili+ | +++ | + |
| N. gonorrhoeae, Orf+, PilC+, Pili- | + | +++ |
| E. coli (E141) | - | - |
| E. coli (E141) + PilC (extern) | - | - |
| E. coli (H2561) | - | - |
| E. coli (H2561) + PilC (extern) | - | - |
| E. coli (H2560) | - | + |
| E. coli (H2560) + PilC (extern) | - | +++ |

Es wurden jeweils drei unabhängige Experimente ausgewertet, wobei die Adhärenz der Neisserien, von jeweils 500 Zellen abgeschätzt und die Adhärenz der E. coli-Stämme pro Epithelzelle gezählt wurde.
+++ 100 %, ++ 50 %, + 25 % Adhärenz.
E. coli E141 = E. coli Stamm HB101 ohne Plasmid; E. coli H2561 = E. coli Stamm HB101 mit Plasmid pBA; E. coli H2560 = E. coli Stamm HB101 mit Plasmid pES25
Das Plasmid pES25 (Figur 1) ist ein pBA-Vektor, der ein ca. 11 kb großes genomisches Fragment aus Neisseria gonorrhoeae enthält, welches die codierenden Bereiche *orfA, orfB* und *orfI* trägt.
Der Stamm E. coli H2560 wurde bei der Deutschen Stammsammlung für Mikroorganismen (DSM, Braunschweig, Deutschland) unter der DSM-Hinterlegungsnummer DSM 10257 hinterlegt.

Das erhaltene Resultat ist überraschend, weil pilustragende Neisserien mit deutlich höherer Affinität an ME-180 Zellen binden, als an Chang Epithelzellen. Dieses Resultat läßt sich dahingehend interpretieren, daß PilC unterschiedliche Adhärenzeigenschaften aufweist, in Abhängigkeit von seiner Lokalisierung. Als Adhäsin-Komponente im Pilus bindet PilC bevorzugt an Rezeptoren der ME-180 Zelloberfläche, während im Komplex mit OrfA als oberflächenlokalisiertes Adhäsin PilC bevorzugt Rezeptoren auf Chang Epithelzellen erkennt. Ob dabei im letzteren Fall Adhäsineigenschaften auch OrfA und/oder OrfB zuzuschreiben sind, ist gegenwärtig nicht bekannt.

Die für rekombinante E. coli-Stämme erzielten Befunde ließen sich mit identischem Resultat in N. gonorrhoeae reproduzieren. Wird der piluslose Stamm N 300 (P- Opa-), der kaum an ME-180 oder Chang Zellen bindet, mit gereinigtem PilC vorinkubiert, so kann hierdurch die Adhärenz an Chang Epithelzellen deutlich verstärkt werden.

Offensichtlich steht mit den beschriebenen experimentellen Ansätzen ein Modell zur Verfügung, das geeignet ist, einen Mechanismus für die Modulation der Adhärenzeigenschaften zu untersuchen, wie sie in kaskadenartiger Abfolge die zunehmend intensive Anheftung der Erreger an Wirtszellen bewirken bzw. dem Gewebetropismus zugrunde liegen können.

## Patentansprüche

1. Nucleinsäure-Molekül oder Fragment davon, das ein Lipoprotein oder biologisch aktives Fragment davon aus Bakterien der Gattung Neisseria codiert, wobei das Lipoprotein oder das Fragment davon die Adhäsion von Neisseria-Zellen an humane Zellen vermittelt, ausgewählt aus der Gruppe bestehend aus
(a) Nucleinsäure-Molekül, das die Sequenz von Nucleotid 583 bis Nucleotid 1545 der unter SEQ ID NO: 1 angegebene Nucleotidsequenz aufweist;
(b) Nucleinsäure-Molekülen, mit der unter SEQ ID NO: 2 dargestellten Nucleotidsequenz;
(c) Nucleinsäure-Molekülen mit der Sequenz von Nucleotid 189 bis Nucleotid 1095 der in SEQ ID NO: 2 dargestellten Nucleotidsequenz;
(d) Nucleinsäure-Molekülen, deren Sequenz aufgrund der Degeneration des genetischen Codes von der Sequenz der unter (a), (b) oder (c) genannten Moleküle abweicht; und
(e) Nucleinsäure-Molekülen, die mit den in (a), (b), (c) oder (d) genannten Molekülen unter stringenten Bedingungen hybridisieren.

2. Nucleinsäure-Molekül nach Anspruch 1, wobei das Molekül aus einer pathogenen Neisseria Spezies stammt.

3. Nucleinsäure-Molekül nach Anspruch 2, wobei die Neisseria Spezies Neisseria gonorrhoeae oder Neisseria meningitidis ist.

4. Nucleinsäure-Molekül nach Anspruch 1, wobei das Lipoprotein oder biologisch aktives Fragment davon die Fähigkeit zur Adhärenz an humane Zellen aufweist.

5. Nucleinsäure-Molekül nach Anspruch 4, wobei das Lipoprotein oder biologisch aktives Fragment in Komplexen mit dem Protein PilC die Fähigkeit zur Adhärenz an humane Zellen besitzt.

6. Vektor enthaltend ein Nucleinsäure-Molekül nach Anspruch 1 bis 5.

7. Vektor nach Anspruch 6, wobei das Nucleinsäure-Molekül mit regulatorischen DNA-Elementen verknüpft ist, die die Expression in pro- oder eukaryontischen Zellen erlauben.

8. Isolierte Wirtszelle, die einen Vektor nach Anspruch 6 oder 7 enthält oder die genetisch manipuliert ist mit einem Nucleinsäure-Molekül nach Anspruch 1 bis 5.

9. Lipoprotein aus Bakterien der Gattung Neisseria, das von einem Nucleinsäure-Molekül nach Anspruch 1, 4 oder 5 codiert wird oder biologisch aktives Fragment davon, wobei das Lipoprotein oder das Fragment davon die Adhäsion von Neisseria-Zellen an humane Zellen vermittelt.

10. Verfahren zur Herstellung des Proteins oder biologisch aktiver Fragmente davon nach Anspruch 9, wobei das Lipoprotein oder das Fragment davon die Adhäsion von Neisseria-Zellen an humane Zellen vermittelt, bei dem Wirtszellen nach Anspruch 8 unter Bedingungen kultiviert werden, die die Expression des Proteins erlauben, und das Protein aus dem Zellen und/oder dem Kulturüberstand isoliert wird.

11. Verfahren zur Isolierung eines Proteins nach Anspruch 9, wobei die Reinigung des Proteins aus Homogenaten von Zellen, die das Protein exprimieren, unter der Ausnutzung der Affinität des Proteins an PilC erfolgt.

12. Antikörper gegen ein Protein oder Fragment davon nach Anspruch 9.

13. Antikörper nach Anspruch 12, wobei dieser gegen die die Adhärenz an humane Zellen vermittelnde Aminosäuresequenz des Proteins gerichtet ist.

14. Arzneimittel enthaltend ein Nucleinsäure-Molekül nach Anspruch 1, 4 oder 5, ein Protein oder biologisch aktives Fragment davon nach Anspruch 9, und/oder Antikörper nach Anspruch 12 oder 13 und gegebenenfalls einen pharmazeutisch verträglichen Träger.

15. Diagnostische Zusammensetzung enthaltend ein Nucleinsäure-Molekül nach Anspruch 1, 4 oder 5, ein Protein oder Fragment davon nach Anspruch 9, und/oder Antikörper nach Anspruch 12 oder 13.

16. Impfstoff, enthaltend ein Nucleinsäure-Molekül nach einem der Ansprüche 1, 4 oder 5, ein Protein oder biologisch aktive Fragmente davon nach Anspruch 9 und/oder einen Antikörper nach Anspruch 12 oder 13.

## Claims

1. Nucleic acid molecule or fragment thereof encoding a lipoprotein or a biologically active fragment thereof from bacteria of the genus Neisseria, wherein the lipoprotein or the fragment thereof mediates the adhesion of Neisseria cells to human cells, selected from the group consisting of
(a) a nucleic acid molecule having the sequence of nucleotide 583 to nucleotide 1545 of the nucleotide sequence shown in SEQ ID NO:1;
(b) nucleic acid molecules with the nucleotide sequence shown in SEQ ID NO:2;
(c) nucleic acid molecules with the sequence of nucleotide 189 to nucleotide 1095 of the nucleotide sequence shown in SEQ ID NO:2;
(d) nucleic acid molecules whose sequence differs from the sequence of the molecules indicated under (a), (b) or (c) due to the degeneration of the genetic code; and
(e) nucleic acid molecules hybridising under stringent conditions with the molecules indicated under (a), (b), (c) or (d).

2. Nucleic acid molecule according to claim 1, wherein the molecule is derived from a pathogenic Neisseria species.

3. Nucleic acid molecule according to claim 2, wherein the Neisseria species is Neisseria gonorrhoeae or Neisseria meningitidis.

4. Nucleic acid molecule according to claim 1, wherein the lipoprotein or the biologically active fragment thereof has the ability to adhere to human cells.

5. Nucleic acid molecule according to claim 4, wherein the lipoprotein or the biologically active fragment in complexes with the protein PilC has the ability to adhere to human cells.

6. Vector containing the nucleic acid molecule according to claims 1 to 5.

7. Vector according to claim 6, wherein the nucleic acid molecule is linked to regulatory DNA elements, which allow the expression in prokaryotic and eukaryotic cells.

8. Isolated host cell, containing the vector according to claim 6 or 7 or which is genetically manipulated with a nucleic acid molecule according to claims 1 to 5.

9. Lipoprotein from bacteria of the genus Neisseria, which is encoded by a nucleic acid molecule according to claim 1, 4 or 5 or a biologically active fragment thereof, wherein the lipoprotein or the fragment thereof mediates the adhesion of Neisseria cells to human cells.

10. Method for the production of the protein or of biologically active fragments thereof according to claim 9, wherein the lipoprotein or the fragment thereof mediates the adhesion of Neisseria cells to human cells, comprising the cultivation of host cells according to claim 8 under conditions allowing the expression of the protein and the isolation of the protein from the cells and/or the culture supernatant.

11. Method for the isolation of a protein according to claim 9, comprising the purification of the protein from homogenates of cells which express the protein using the affinity of the protein for PilC.

12. Antibody against a protein or fragment thereof according to claim 9.

13. Antibody according to claim 12, wherein the antibody is directed against the amino acid sequence of the protein which mediates the adherence to human cells.

14. Pharmaceutical composition containing a nucleic acid molecule according to claim 1, 4 or 5, a protein or a biologically active fragment thereof according to claim 9 and/or antibodies according to claim 12 or 13 and optionally a pharmaceutically acceptable carrier.

15. Diagnostic composition containing a nucleic acid molecule according to claim 1, 4 or 5, a protein or fragment thereof according to claim 9 and/or antibodies according to claim 12 or 13.

16. Vaccine containing a nucleic acid molecule according to any one of claims 1, 4 or 5, a protein or biologically active fragments thereof according to claim 9 and/or an antibody according to claim 12 or 13.

## Revendications

1. Molécule d'acide nucléique ou fragment de celle-ci qui code une lipoprotéine ou un fragment biologiquement actif de celle-ci provenant de bactéries du genre Neisseria, dans laquelle la lipoprotéine ou le fragment de celle-ci permet l'adhésion des cellules Neisseria aux cellules humaines, sélectionnée dans le groupe consistant en :
a. une molécule d'acide nucléique qui présente la séquence du nucléotide 583 au nucléotide 1545 de la séquence de nucléotides donnée sous SEQ ID NO : 1 ;
b. des molécules d'acides nucléiques comportant la séquence de nucléotides représentée sous SEQ ID NO : 2 ;
c. des molécules d'acides nucléiques comportant la séquence du nucléotide 189 au nucléotide 1095 de la séquence de nucléotides représentée dans SEQ ID NO : 2 ;
d. des molécules d'acides nucléiques dont la séquence, en raison de la dégénérescence du code génétique, se différencie de la séquence des molécules citées sous (a), (b) ou (c) ; et
e. des molécules d'acides nucléiques qui s'hybrident avec les molécules citées dans (a), (b), (c) ou (d) dans des conditions stringentes.

2. Molécule d'acide nucléique selon la revendication 1, dans laquelle la molécule provient d'une espèce Neisseria pathogène.

3. Molécule d'acide nucléique selon la revendication 2, dans laquelle l'espèce Neisseria est Neisseria gonorrhoeae ou Neisseria meningitidis.

4. Molécule d'acide nucléique selon la revendication 1, dans laquelle la lipoprotéine ou le fragment biologiquement actif de celle-ci présente la capacité à adhérer aux cellules humaines.

5. Molécule d'acide nucléique selon la revendication 4, dans laquelle la lipoprotéine ou le fragment biologiquement actif possède, dans des complexes avec la protéine PilC, la capacité à adhérer aux cellules humaines.

6. Vecteur contenant une molécule d'acide nucléique selon la revendication 1 à 5.

7. Vecteur selon la revendication 6, dans lequel la molécule d'acide nucléique est liée à des éléments d'ADN régulateurs, qui permettent l'expression dans des cellules procaryotes ou eucaryotes.

8. Cellule hôte isolée qui contient un vecteur selon la revendication 6 ou 7 ou qui est manipulée génétiquement avec une molécule d'acide nucléique selon la revendication 1 à 5.

9. Lipoprotéine provenant de bactéries du genre Neisseria, qui est codée par une molécule d'acide nucléique selon la revendication 1, 4 ou 5 ou un fragment biologiquement actif de celle-ci, la lipoprotéine ou le fragment de celle-ci permettant l'adhésion des cellules Neisseria aux cellules humaines.

10. Procédé pour la production de protéines ou fragments biologiquement actifs de celles-ci selon la revendication 9, dans lequel la lipoprotéine et le fragment de celle-ci permettent l'adhésion des cellules Neisseria aux cellules humaines, par lequel procédé des cellules hôtes selon la revendication 8 sont cultivées dans des conditions qui permettent l'expression de la protéine, et la protéine est isolée de la cellule et/ou du surnageant de culture.

11. Procédé pour l'isolement d'une protéine selon la revendication 9, dans lequel la purification de la protéine provenant des homogénats de cellules exprimant la protéine est réalisée en exploitant l'affinité de la protéine pour PilC.

12. Anticorps contre une protéine ou un fragment de celle-ci selon la revendication 9.

13. Anticorps selon la revendication 12, dans lequel celui-ci est dirigé contre la séquence d'acides aminés permettant l'adhérence aux cellules humaines.

14. Médicament contenant une molécule d'acide nucléique selon la revendication 1, 4 ou 5, une protéine ou un fragment biologiquement actif de celle-ci selon la revendication 9 et/ou des anticorps selon la revendication 12 ou 13 et, le cas échéant, un véhicule pharmaceutiquement compatible.

15. Composition de diagnostic contenant une molécule d'acide nucléique selon la revendication 1, 4 ou 5, une protéine ou un fragment de celle-ci selon la revendication 9 et/ou des anticorps selon la revendication 12 ou 13.

16. Vaccin contenant une molécule d'acide nucléique selon l'une quelconque des revendications 1, 4 ou 5, une protéine ou des fragments biologiquement actifs de celle-ci selon la revendication 9, et/ou un anticorps selon la revendication 12 ou 13.
